(19) Europäisches Patentamt / European Patent Office / Office européen des brevets

(11) EP 1 556 386 B1

(12) **FASCICULE DE BREVET EUROPEEN**

(45) Date de publication et mention de la délivrance du brevet:
**15.12.2010 Bulletin 2010/50**

(21) Numéro de dépôt: **03782510.6**

(22) Date de dépôt: **28.10.2003**

(51) Int Cl.:
*C07D 493/10* (2006.01) *A61K 49/00* (2006.01)
*G01B 21/00* (2006.01)

(86) Numéro de dépôt international:
**PCT/FR2003/003205**

(87) Numéro de publication internationale:
**WO 2004/039810 (13.05.2004 Gazette 2004/20)**

(54) **PHTHALEINES DE PURETE ELEVEE ET LEUR PROCEDE DE PREPARATION**

PHTHALEINE MIT HÖHEREM REINHEITSGRAD UND IHR VERFAHREN ZUR HERSTELLUNG

HIGH PURITY PHTHALEIN DERIVATIVES AND METHOD FOR PREPARING SAME

(84) Etats contractants désignés:
**AT BE BG CH CY CZ DE DK EE ES FI FR GB GR HU IE IT LI LU MC NL PT RO SE SI SK TR**
Etats d'extension désignés:
**LT LV**

(30) Priorité: **29.10.2002 FR 0213528**

(43) Date de publication de la demande:
**27.07.2005 Bulletin 2005/30**

(73) Titulaire: **Patent Pharma**
**75020 Paris (FR)**

(72) Inventeurs:
- **TRAN-GUYON, Joanne**
**F-78180 Montigny le Bretonneux (FR)**
- **SCHERNINSKI, François**
**F-75011 Paris (FR)**

(74) Mandataire: **Touati, Catherine**
**Cabinet Plasseraud**
**52 Rue de la Victoire**
**75440 Paris Cedex 09 (FR)**

(56) Documents cités:
**DE-C- 360 691** **US-A- 1 931 049**
**US-A- 1 965 842** **US-A- 5 637 733**

- **R.C. GIBBS: "The absorption spectra of some phthaleins of the trihydroxyphenols" J. AM. CHEM. SOC, vol. 51, 1929, page 1755-66, XP002248840**
- **SHAWN C. BURDETTE ET AL.: "fluorescent sensors for Zn2+ Based on a Fluorescein Platform: Synthesis, Properties and Intracellular Distribution" J. AM. CHEM. SOC, vol. 123, 2001, page 7831-41, XP002248841**
- **DATABASE CA [Online] CHEMICAL ABSTRACTS SERVICE, COLUMBUS, OHIO, US; XP002248842 accession no. STN Database accession no. 91: 124850 -& MATVEETS, M.A. ET AL.: "Study of spectrophotometric and luminescence properties of hydroxyxanthene dyes in aqueous soution" ZHURNAL ANALITICHESKOI KHIMII, vol. 34, no. 6, 1979, pages 1049-54, XP008020063**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 556 386 B1

Printed by Jouve, 75001 PARIS (FR)

## Description

La présente invention porte sur des phtaléines de pureté élevée et sur leur procédé de préparation. Elle porte plus particulièrement sur la fluorescéine de pureté élevée.

Dans la présente description on entend par « phtaléine de pureté élevée » une phtaléine contenant au plus 1% en poids, de préférence au plus 0,5% en poids d'impuretés.

Les phtaléines sont des molécules présentant le motif xanthène suivant :

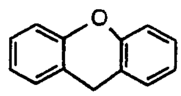

Ces produits sont utiles comme colorants dans diverses industries, notamment l'industrie textile, l'industrie du papier, l'imprimerie, la reprographie, l'industrie alimentaire, l'industrie cosmétique et l'industrie pharmaceutique. Les phtaléines font, actuellement, l'objet d'un important regain d'intérêt dans le domaine de la santé, pour leur utilisation diagnostique, notamment dans le cadre de l'imagerie médicale et dans le domaine des biotechnologies pour le marquage des molécules biologiques (acides nucléiques, protéines, lipoprotéines, lipides membranaires) et le suivi de l'activité biochimique intracellulaire ou extracellulaire des molécules biologiques.

Par exemple, la fluorescéine est une phtaléine couramment utilisée en ophtalmologie pour la réalisation d'angiographies en fluorescence de la rétine. L'intérêt diagnostique de l'angiographie fluorescéinique est actuellement accru par l'apparition de nouveaux médicaments permettant le traitement des pathologies vasculaires de la rétine et de la choroïde et la disponibilité d'une nouvelle génération de rétinographes permettant la réalisation d'une imagerie numérisée en fluorescence plus performante et résolutive que les anciens systèmes d'acquisition sur émulsions photographiques.

Parallèlement les exigences de qualité et de sécurité des nouvelles normes pharmaceutiques internationales (ICH : International Commission of Harmonisation, ICH Q Topic Q3A 1999) se sont considérablement accrues. Il en est de même en ce qui concerne les applications des phtaléines dans le domaine des biotechnologies qui nécessitent des réactifs de qualité de plus en plus élevée. Pour répondre aux exigences actuelles, le niveau de pureté des phtaléines utilisées dans le domaine pharmaceutique ou celui des biotechnologies doit nécessairement être très élevé. A titre d'exemple, dans la publication « Effective differences in the formulation of intravenous fluorescein and related side effects », Am. J. Ophtalmol. 78,2 : 217-221, 1974, L. Yannuzzi a montré qu'il existe une corrélation entre la pureté des phtaléines, en particulier celle de la fluorescéine, et la tolérance de ces substances lorsqu'elles sont administrées par voie injectable chez l'homme.

Des phtaléines présentent la formule générale (I) :

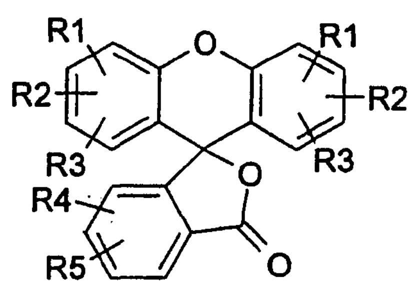

(I)

dans laquelle R1, R2, R3, R4, R5 qui sont identiques ou différents les uns des autres, sont choisis dans le groupe comprenant les radicaux ou groupements suivants : hydrogène, hydroxyle, halogène, acétyle, amino, phosphate, nitro, sulfonate, carboxy, alkyl-carboxy de 2 à 30 atomes de carbone, alkyle ayant de 1 à 30 atomes de carbone, cycloalkyle ayant de 3 à 12 atomes de carbone, alkyloxy ayant de 1 à 30 atomes de carbone, halogénoalkyle ayant de 1 à 30 atomes de carbone, hydroxyalkyle ayant de 1 à 30 atomes de carbone, alkylester ayant de 2 à 40 atomes de carbone, nitroalkyle ayant de 1 à 30 atomes de carbone, carboxyalkyle ayant de 2 à 30 atomes de carbone, aminoalkyle ayant

de 1 à 30 atomes de carbone, sulfoalkyle ayant de 1 à 30 atomes de carbone, aryle, aryloxy, aryl-alkyle, halogénoaryle, arylester, succinimidylester, isothiocyanate, maléimide, iodoacétamide, halogénoacétamide, chlorosulfonique, les bases puriques ou pyrimidiques, les oses, de préférence les hexoses ou les pentoses, les oligosides et les polyosides, les polypeptides, les protéines et les phospholipides,

R3 et R5 ne représentant pas chacun l'hydrogène lorsque R1 représente un groupe $-CH_2-CH_2-COOH$, R2 représente un groupe hydroxy et R4 représente un groupe -COOH,

ces phtaléines ne contenant pas plus de 1% en poids, de préférence pas plus de 0,5% en poids et plus préférentiellement encore pas plus de 0,2% en poids d'impuretés résiduelles.

Une phtaléine particulièrement intéressante, notamment pour des applications ophtalmiques est la fluorescéine présentant une telle pureté.

Il est connu de préparer les phtaléines de formule (I) par condensation d'un dérivé de l'anhydride phtalique et d'un dérivé phénolique ayant une positon libre en ortho d'un groupe hydroxyle (voir notamment US-A-5,637,733 et Shawn C. Burdette et al., J.Am.Chem.Soc., vol.123, 2001, pages 7831-41).

Cette condensation est réalisée par chauffage à la température de fusion d'un mélange de l'anhydride phtalique et du dérivé phénolique, dans les proportions souhaitées.

Cette condensation peut éventuellement être réalisée dans un solvant de dilution. Elle peut également être conduite en présence d'un catalyseur.

En l'absence de solvant, lors du chauffage, le milieu réactionnel s'épaissit rapidement et a tendance à prendre en masse. Au sein du milieu réactionnel, il se forme alors des zones où la température est trop élevée et des zones où la température n'est pas assez élevée.

Dans les zones où la température est trop élevée, les réactifs et/ou le produit de réaction sont dégradés et dans les zones où la température n'est pas assez élevée, la réaction n'est pas complète. Le produit obtenu est de médiocre qualité car il comporte des sous-produits très difficiles à éliminer.

Afin d'améliorer ce procédé connu, on a proposé l'ajout d'un solvant inerte ou l'utilisation d'un catalyseur.

Aussi, le brevet US 1,931,049 décrit l'ajout au milieu réactionnel d'un solvant inerte constitué par un hydrocarbure benzénique ou aliphatique, plus particulièrement l'orthodichlorobenzène. Cependant, la réaction de condensation est alors incomplète, et il se forme des produits intermédiaires difficiles à éliminer par la suite. Il s'en suit que le procédé décrit dans le brevet US 1,931,049 ne permet pas l'obtention d'une phtaléine de pureté élevée selon la définition de la présente demande. Par ailleurs, les hydrocarbures aliphatiques supérieurs ne sont pas miscibles avec le milieu réactionnel; par conséquent, ils n'apportent pas d'amélioration sur le plan du transfert thermique et gênent l'élimination de l'eau formée ce qui ralentit la réaction, laquelle a lieu exclusivement en milieu hydrophobe.

Pour ce qui est des catalyseurs proposés pour améliorer le rendement de la réaction, il s'agit de l'acide sulfurique concentré, du chlorure de zinc anhydre et du chlorure d'étain.

Dans le brevet allemand DE 360691, il a été proposé d'utiliser en tant que catalyseur, un acide sulfonique aromatique, tel que l'acide benzène sulfonique, seul ou associé à l'un des trois catalyseurs précités.

Il se trouve toutefois que l'adjonction de ces catalyseurs entraîne une prise en masse rapide du produit de la réaction avec un emprisonnement des impuretés qui ne peuvent plus être éliminées du produit recherché.

Afin d'éliminer les sous-produits formés et les impuretés, des procédés d'isolement et de purification ont été mis au point mais n'ont pas permis d'améliorer sensiblement la qualité des phtaléines.

Un procédé classiquement utilisé consiste à alcaliniser la phtaléine en milieu aqueux pour la dissoudre puis à l'acidifier pour permettre sa précipitation. Ces deux étapes sont renouvelées successivement pour tenter d'éliminer les impuretés. Néanmoins, cette méthode ne permet pas une amélioration notable de la pureté du produit car si les impuretés se dissolvent avec la phtaléine au cours de l'étape d'alcalinisation, elles précipitent à nouveau en même temps que la phtaléine au cours de l'étape d'acidification. De plus, cette méthode de purification a pour inconvénient de produire une quantité importante de sels qui sont difficiles et coûteux à éliminer par la suite. Un tel procédé de purification est notamment décrit dans le document DD136498.

Le brevet US 1,965,842 décrit la purification des phtaléines, dérivées de l'hydroxybenzène, par une extraction directe des sous-produits à l'aide de dichlorobenzène utilisé seul ou en mélange avec d'autres solvants. Cette extraction directe du produit brut avec le solvant en question ne permet cependant pas une élimination poussée des impuretés qui restent en partie piégées dans les cristaux de phtaléine.

Aucun des procédés de purification décrits dans l'art antérieur ne permet d'atteindre un niveau de pureté suffisant pour permettre un usage pharmaceutique des phtaléines.

Etant donné l'intérêt de ces molécules pour le diagnostic médical, la fabrication de phtaléines de pureté élevée correspond à un réel besoin médical et répondrait à une forte demande, notamment pour des applications pharmaceutiques en ophtalmologie, pour le diagnostic notamment en imagerie médicale ou dans le domaine des applications en biotechnologie (par exemple pour le marquage de molécules).

Les inventeurs, à l'issue de recherches approfondies, ont eu le mérite de trouver qu'il était possible d'obtenir des produits de pureté élevée par condensation d'un anhydride phtalique avec un dérivé phénolique ou naphtolique

dans un solvant particulier qui est constitué par un ester d'acide organique.

Ils ont également trouvé que l'utilisation des esters d'acide organique comme solvants permettait de réaliser cette condensation, avec un excellent rendement, supérieur à 75%.

En effet, ces solvants ont la particularité

- d'une part, de conduire à une cristallisation spécifique de la phtaléine issue de la condensation et d'exclure, des cristaux formés, toutes les impuretés qui restent dissoutes dans le milieu réactionnel et
- d'autre part, de permettre que la réaction de condensation soit totale et prédomine au détriment des réactions secondaires indésirables, ce qui conduit à la consommation totale des réactifs et minimise la formation des sous-produits.

Le procédé conforme à l'invention permet ainsi d'obtenir des phtaléines de pureté élevée avec un rendement satisfaisant sur le plan industriel.

Plus particulièrement, l'invention a donc pour objet un procédé de préparation de phtaléines, débarrassées de leurs impuretés résiduelles, présentant la formule générale (I) :

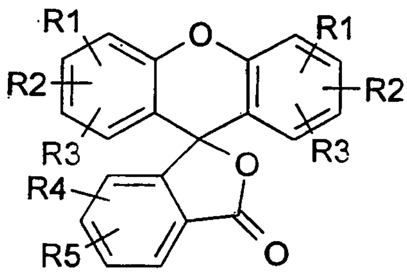

(I) .

dans laquelle R1, R2, R3, R4, R5 qui sont identiques ou différents les uns des autres, sont choisis dans le groupe comprenant les radicaux ou groupements suivants : hydrogène, hydroxyle, halogène, acétyle, amino, phosphate, nitro, sulfonate, carboxy, alkyl-carboxy de 2 à 30 atomes de carbone, alkyle ayant de 1 à 30 atomes de carbone, cycloalkyle ayant de 3 à 12 atomes de carbone, alkyloxy ayant de 1 à 30 atomes de carbone, halogénoalkyle ayant de 1 à 30 atomes de carbone, hydroxyalkyle ayant de 1 à 30 atomes de carbone, alkylester ayant de 2 à 40 atomes de carbone, nitroalkyle ayant de 1 à 30 atomes de carbone, carboxyalkyle ayant de 2 à 30 atomes de carbone, aminoalkyle ayant de 1 à 30 atomes de carbone, sulfoalkyle ayant de 1 à 30 atomes de carbone, aryle, aryloxy, aryl-alkyle, halogénoaryle, arylester, succinimidylester, isothiocyanate, maléimide, iodoacétamide, halogénoacétamide, chlorosulfonique, les bases puriques ou pyrimidiques, les oses, de préférence les hexoses ou les pentoses, les oligosides et les polyosides, les polypeptides, les protéines et les phospholipides,
R3 et R5 ne représentant pas chacun l'hydrogène lorsque R1 représente un groupe $-CH_2-CH_2-COOH$, R2 représente un groupe hydroxy et R4 représente un groupe -COOH,
par condensation d'un dérivé de l'anhydride phtalique de formule (II) avec un composé phénolique ou naphtolique de formule (III)

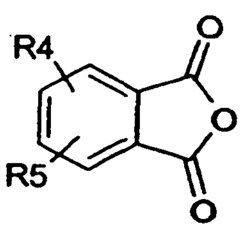

(II)

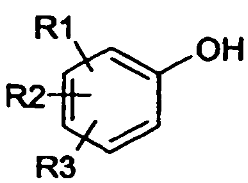

(III)

dans lesquelles R1, R2, R3, R4, R5 ont les mêmes significations que ci-dessus,
au sein d'un solvant constitué par un ester d'acide organique.

De façon particulièrement avantageuse, le composé de départ (III) qui est condensé avec l'anhydride phtalique (II) est choisi dans le groupe comprenant notamment le résorcinol, l'orcinol, le naphtol, le pyrogallol, l'alkylaminophénol et l'arylaminophénol.

Dans le cas où le résorcinol est utilisé comme produit de départ, le procédé conforme à l'invention permet la préparation de la fluorescéine.

De façon avantageuse, le solvant utilisé dans le procédé conforme à l'invention est un ester d'acide organique de formule (IV)

$$R_6\text{-}COOR_7 \qquad (IV)$$

dans laquelle $R_6$ est choisi dans le groupe comprenant les radicaux ou groupements suivants : hydrogène, alkyle ayant de 1 à 30 atomes de carbone, cycloalkyle ayant de 3 à 12 atomes de carbone, halogénoalkyle ayant de 1 à 30 atomes de carbone, hydroxyalkyle ayant de 1 à 30 atomes de carbone, nitroalkyle ayant de 1 à 30 atomes de carbone, aryle, aryloxy, alkyl-aryle, aryl-alkyle, aryl-alkyle substitué, halogénoaryle, arylester, alkylester ayant de 2 à 40 atomes de carbone, alkyloxy ayant de 1 à 30 atomes de carbone, $R_7$ représentant un groupe alkyle ayant de 1 à 30 atomes de carbone, cycloalkyle ayant de 3 à 12 atomes de carbone, halogénoalkyle ayant de 1 à 30 atomes de carbone, hydroxyalkyle ayant de 1 à 30 atomes de carbone, nitroalkyle ayant de 1 à 30 atomes de carbone, aryle, aryloxy, alkyl-aryl, aryl-alkyle, aryl-alkyle substitué, halogénoaryle, arylester, alkylester ayant de 2 à 40 atomes de carbone, alkyloxy ayant de 1 à 30 atomes de carbone.

De façon particulièrement avantageuse, le susdit solvant est choisi dans le groupe comprenant les benzoates, les heptanoates, les octanoates, les laurates, les myristates et les palmitates de méthyle, d'éthyle, de propyle ou de butyle, et leurs mélanges.

Le solvant est choisi en fonction de sa température d'ébullition, de façon à permettre de conduire la réaction de condensation à une température comprise entre 150°C et 250°C.

La réaction de condensation peut être conduite à la pression atmosphérique ou sous une pression adaptée en fonction de la différence existant entre la température correspondant au point d'ébullition du solvant utilisé et la température nécessaire pour conduire la réaction, en particulier sous une pression supérieure à la pression atmosphérique lorsque le point d'ébullition du composé(IV)est plus bas que la température de la réaction.

La réaction de condensation peut être conduite en présence d'un catalyseur choisi dans le groupe comprenant notamment les acides de Lewis, tels que $ZnCl_2$ ou $AlCl_3$, les acides de Brönsted tels que $H_2SO_4$, l'acide polyphosphorique ou leurs sels.

De façon avantageuse, le catalyseur utilisé est un sel alcalin d'hydrogénosulfate. De façon particulièrement avantageuse, le catalyseur utilisé est l'hydrogénosulfate de potassium ($KHSO_4$) ou l'hydrogénosulfate de sodium. L'utilisation d'hydrogénosulfate comme catalyseur permet l'obtention d'un excellent rendement pour la réaction de condensation et présente l'avantage, contrairement aux autres catalyseurs, de permettre l'obtention d'une condensation complète des réactifs, de pouvoir être éliminé totalement de la phtaléine obtenue et de ne pas induire la formation de goudrons dans le milieu réactionnel.

A l'issue de la réaction de condensation, on obtient un produit brut dont la pureté organique est déjà beaucoup plus élevée que celle des produits issus des procédés de l'art antérieur puisqu'elle est égale ou supérieure à 95%.

Néanmoins cette pureté n'est pas encore suffisante pour permettre une utilisation pharmaceutique notamment par voie injectable. D'autre part, comme montré ci-dessus, les méthodes de purification décrites dans l'art antérieur ne conduisent pas à un procédé permettant d'améliorer notablement cette pureté.

A l'issue de recherches approfondies et de façon tout à fait inattendue, les inventeurs ont trouvé qu'il était possible d'augmenter considérablement la pureté des phtaléines brutes obtenues par la réaction de condensation en les traitant par un acide fort ou un de ses précurseurs dans un milieu organique anhydre.

Selon un mode de réalisation avantageux, le procédé conforme à l'invention comporte, par conséquent, après la réaction de condensation, une étape consistant à acidifier le produit résultant de la condensation, en milieu organique anhydre, par addition d'un acide fort ou un de ses précurseurs choisi dans le groupe comprenant notamment l'acide sulfurique, l'acide chlorhydrique, l'acide bromhydrique, l'acide fluorhydrique, l'acide iodhydrique, l'acide polyphosphorique, le pyrophosphate ($P_2O_5$), et leurs mélanges. Cette acidification est conduite jusqu'à la conversion des cristaux de phtaléine résultant de la condensation, en cristaux de phtaléine de structure différente.

Cette étape d'acidification a pour effet de convertir la phtaléine brute insoluble et dispersée dans le milieu organique en une forme de structure cristalline différente et peu soluble dans ce même milieu. Cette conversion est très rapide, et s'effectue en passant par une phase de solubilisation intermédiaire au cours de laquelle les impuretés sont libérées et éliminées totalement des cristaux de phtaléine. Ce nouveau procédé de purification est très avantageux car il nécessite très peu de solvant et permet l'obtention d'une pureté très élevée en un temps très bref.

Selon le procédé de la présente invention, cette étape de purification est réalisée en dispersant la phtaléine brute issue de la condensation, dans un solvant anhydre, de préférence dans un alcool, une cétone, un éther, un solvant halogéné, utilisé seul ou en mélange, plus préférentiellement encore dans l'éthanol absolu ou l'acétone seul ou en mélange.

La .dispersion de phtaléine brute ainsi obtenue est acidifiée par addition d'un acide fort ou un de ses précurseurs choisi dans le groupe comprenant notamment l'acide sulfurique, l'acide chlorhydrique, l'acide bromhydrique, l'acide fluorhydrique, l'acide iodhydrique, l'acide polyphosphorique, le pyrophosphate ($P_2O_5$) et leurs mélange.

Selon un mode de réalisation particulièrement avantageux, l'acidification est réalisée soit en faisant barboter de l'acide chlorhydrique gazeux dans la dispersion de phtaléine, soit en ajoutant de l'acide chlorhydrique préalablement dissous dans un solvant organique.

Selon un autre mode de réalisation avantageux du procédé conforme à l'invention, le produit obtenu après acidification est lavé à l'aide d'une solution de lavage choisie dans le groupe comprenant l'eau, les solvants polaires comme les alcools, les cétones, ou les solvants légèrement polaires comme les éthers et les solvants halogénés, utilisés purs ou en mélange.

A l'issue de l'étape de purification, les phtaléines ainsi traitées ont une pureté supérieure à 98%, de préférence supérieure à 99 %, plus préférentiellement supérieure à 99,5%, et encore plus préférentiellement de 99,8%.

Les phtaléines ainsi obtenues ont une qualité adaptée pour permettre la préparation d'une autre phtaléine de formule (I) par modification chimique des groupements R1, R2, R3, R4, R5 selon les procédés classiques de l'art, notamment pour l'obtention de phtaléines utilisables pour le marquage des molécules biologiques (acides nucléiques, protéines, lipoprotéines, lipides membranaires) et dans le domaine des applications en biotechnologie (par exemple pour le marquage de molécules et de leur activité biochimique intra ou extracellulaire).

L'invention est particulièrement adaptée pour la préparation de fluorescéine de très grande pureté, c'est à dire telle que sa teneur en chacun des sous-produits de la réaction est inférieure ou égale à 0,2% et de préférence inférieure ou égale à 0,1%, la somme des teneurs en chacun de ces sous-produits étant inférieure ou égale à 0,5%.

Le procédé de préparation d'une fluorescéine présentant la susdite pureté comprend les étapes successives suivantes :

- condensation de l'anhydride phtalique avec le résorcinol, dans un solvant qui est un ester d'acide organique aliphatique ou aromatique, de préférence le benzoate de méthyle, en présence d'un catalyseur choisi dans le groupe des acides de Brönsted,
- mise en suspension des cristaux de couleur rouge obtenus à l'étape précédente dans un solvant anhydre choisi dans le groupe comprenant les alcools tels que l'éthanol absolu, les cétones-telles que l'acétone, les éthers, les solvants halogénés, ou leurs mélanges,
- acidification de la suspension obtenue par addition d'un acide fort ou un de ses précurseurs choisi dans le groupe comprenant l'acide sulfurique, l'acide chlorhydrique, l'acide bromhydrique, l'acide fluorhydrique, l'acide iodhydrique, l'acide polyphosphorique, le pyrophosphate ($P_2O_5$), ou leurs mélanges, jusqu'à l'obtention de cristaux de couleur jaune,
- lavage des cristaux ainsi obtenus à l'aide d'une solution de lavage choisie dans le groupe comprenant l'eau, les alcools, les cétones, les éthers et les solvants halogénés, purs ou en mélange, jusqu'à l'obtention de cristaux de couleur rouge.

Les cristaux de fluorescéine de couleur rouge présentent un cristallographe RX tel que celui représenté sur la figure 1; les cristaux de fluorescéine de couleur jaune présentent un cristallographe RX tel que celui représenté sur la figure 2; ces cristallographes étant réalisés sur l'appareillage suivant: Générateur PHILIPS 1729, Goniomètre PHILIPS 1050, Radiation Cu K$\alpha$, Logiciel acquisition GONIO et Logiciel traitement RAYON, dans les conditions opératoires suivantes:

| | |
|---|---|
| Tension | : 40 mV |
| Intensité | : 40 mA |
| Nombre de points | : 4000 |
| Nombre de passages | : 10 |
| Période d'acquisition | : 250 ms |
| Angle de départ (˚$\theta$) | : 3.000 |
| Angle de fin (˚$\theta$) | : 23.000 |
| Etalon | : Silicium |

Selon un mode de réalisation avantageux du procédé de préparation de la fluorescéine, le catalyseur utilisé pour la réaction de condensation est de l'hydrogénosulfate d'un métal alcalin, de préférence de l'hydrogénosulfate de potassium ou de sodium.

Selon un autre mode de réalisation avantageux du procédé de préparation de la fluorescéine, l'acidification est réalisée par barbotage d'acide chlorhydrique gazeux dans la suspension de fluorescéine ou par l'action, sur cette fluorescéine, d'acide chlorhydrique en solution dans un solvant organique anhydre, choisi de préférence dans le groupe comprenant les alcools, les cétones, les éthers et les solvants halogénés utilisés isolément ou en mélange, plus préférentiellement encore, l'isopropanol, l'éthanol absolu ou l'acétone, purs ou en mélange.

De façon avantageuse, l'étape de lavage est réalisée à l'aide d'un mélange d'eau, d'éthanol et d'acétone.

Grâce à ce procédé conforme à l'invention, il est possible de préparer de la fluorescéine présentant une pureté égale ou supérieure à 99,7%, ce qui présente des avantages incontestables notamment pour les utilisations pharmaceutiques en diagnostic notamment en imagerie médicale ou encore dans le domaine des applications en biotechnologie.

Les inventeurs ont mis en évidence une nouvelle forme cristallographique de la fluorescéine présentant des cristaux de couleur jaune. Cette nouvelle forme cristalline est identifiée par le spectre de radiocristallographie de la Figure 2 qui a été réalisé dans les conditions mentionnées ci-dessus.

Ils ont également mis en évidence une nouvelle forme cristallographique des composés suivants 4',5'-dihydroxyfluorescéine et 4',5'-diméthylfluorescéine.

Ainsi, l'invention porte sur les cristaux de fluorescéine de couler jaune dont le spectre de radiocristallographie est donné sur la Figure 2.

L'invention porte également sur les cristaux de 4',5'-dihydroxyfluorescéine de couleur jaune dont le spectre de radiocristallographie est donné sur la Figure 4.

Elle porte également sur les cristaux de 4',5'-diméthylfluorescéine de couleur rouge sombre dont le spectre de radiocristallographie est donné sur la Figure 6.

Tous les spectres ont été réalisés avec l'appareillage et dans les conditions mentionnés ci-dessus.

L'invention va être décrite de façon plus détaillée à l'aide des exemples suivants qui ne sont pas limitatifs mais relatifs à des modes de réalisation avantageux.

**EXEMPLES**

**EXEMPLE 1: Préparation de fluorescéine de pureté élevée**

**Synthèse de la fluorescéine** :

Un mélange comprenant 55 g de résorcinol, 30 g d'anhydride phtalique, 2 g d'hydrogénosulfate de potassium et 500 ml de benzoate de méthyle est porté à 200°C pendant 6 heures. Après refroidissement, les cristaux rouges de fluorescéine brute sont lavés à l'acétone et séchés.

Masse obtenue = 51,8 g (78%).

Ces cristaux sont analysés par radiocristallographie dans les conditions opératoires suivantes:

Appareil

Générateur PHILIPS 1729
Goniomètre PHILIPS 1050
Radiation Cu Kα
Logiciel acquisition GONIO
Logiciel traitement RAYON

Conditions

| | |
|---|---|
| Tension | : 40 mV |
| Intensité | : 40 mA |
| Nombre de points | : 4000 |
| Nombre de passages | : 10 |
| Période d'acquisition | : 250 ms |
| Angle de départ (°θ) | : 3.000 |
| Angle de fin (°θ) | : 23.000 |
| Etalon | : Silicium |

On obtient le radiocristallographe de la figure 1, dont les pics sont identifiés ci-après.

| Thêta (degrés) | Distance (Å) | % intensités | Nombre de coups |
|---|---|---|---|
| 5.3600 | 8.2457 | 18.09 | 647 |
| 5.9500 | 7.4306 | 31.10 | 1112 |
| 6.7000 | 6.6020 | 36.30 | 1298 |
| 8.3750 | 5.2883 | 45.95 | 1643 |
| 9.1050 | 4.8675 | 10.79 | 386 |
| 9.4300 | 4.7012 | 14.09 | 504 |
| 9.9950 | 4.4379 | 8.03 | 287 |
| 10.9150 | 4.0678 | 9.73 | 348 |
| 11.6200 | 3.8241 | 16,41 | 587 |
| 11.7300 | 3.7888 | 5.40 | 193 |
| 12.4200 | 3.5813 | 2.82 | 101 |
| 13.2150 | 3.3694 | 100.00 | 3576 |
| 13.5050 | 3.2983 | 7.47 | 267 |
| 13.8850 | 3.2097 | 79.03 | 2826 |
| 14.9650 | 2.9828 | 5.62 | 201 |
| 15.3100 | 2.9172 | 3.91 | 140 |
| 15.7600 | 2.8359 | 11.69 | 418 |
| 16.0000 | 2.7945 | 8.67 | 310 |
| 16.9100 | 2.6481 | 4.28 | 153 |
| 17.4750 | 2.5650 | 3.97 | 142 |
| 17.8550 | 2.5122 | 3.80 | 136 |
| 18.2300 | 2.4622 | 2.52 | 90 |
| 18.5150 | 2.4256 | 2.96 | 106 |
| 18.8250 | 2.3871 | 5.03 | 180 |
| 19.0800 | 2.3563 | 3.27 | 117 |
| 20.4300 | 2.2066 | 3.05 | 109 |
| 20.6800 | 2.1811 | 4.53 | 162 |
| 20.9300 | 2.1562 | 3.08 | 110 |
| 21.1800 | 2.1319 | 2.38 | 85 |
| 22.9400 | 1.9762 | 4.53 | 162 |
| longueur d'onde: 1.54051 Å | | | |

**Purification de la fluorescéine brute :**

50 g de fluorescéine brute obtenus à l'étape précédente sont agités dans 1000 ml de mélange éthanol/acétone. A ce mélange est ajouté de l'acide sulfurique concentré jusqu'à conversion complète des cristaux rouges en cristaux jaunes.

Les cristaux jaunes obtenus sont analysés par radiocristallographie comme précédemment. On obtient le radiocristallographe de la figure 2, dont les pics sont identifiés ci-après.

| Thêta (degrés) | Distances (Å) | % Intensités | Nbre de coups |
|---|---|---|---|
| 3.3500 | 13.1813 | 8.55 | 56 |
| 4.0050 | 11.0283 | 47.18 | 309 |
| 4.8550 | 9.1010 | 12.98 | 85 |
| 5.2650 | 8.3940 | 9.01 | 59 |
| 5.4500 | 8.1099 | 11.45 | 75 |
| 5.7900 | 7.6352 | 52.98 | 347 |
| 6.3000 | 7.0193 | 16.95 | 111 |
| 6.7700 | 6.5340 | 17.86 | 117 |
| 7.4900 | 5.9090 | 17.56 | 115 |

(suite)

| Thêta (degrés) | Distances (Å) | % Intensités | Nbre de coups |
|---|---|---|---|
| 8.0300 | 5.5140 | 69.77 | 457 |
| 8.4250 | 5.2572 | 33.13 | 217 |
| 8.7800 | 5.0462 | 8.40 | 55 |
| 9.1550 | 4.8411 | 15.88 | 104 |
| 9.3400 | 4.7461 | 42.44 | 278 |
| 9.6550 | 4.5926 | 34.66 | 227 |
| 9.8800 | 4.4891 | 24.43 | 160 |
| 10.1950 | 4.3518 | 57.71 | 378 |
| 10.5800 | 4.1951 | 58.02 | 380 |
| 10.7350 | 4.1352 | 38.17 | 250 |
| 11.1650 | 3.9779 | 28.85 | 189 |
| 11.4800 | 3.8701 | 30.23 | 198 |
| 11.7200 | 3.7919 | 59.85 | 392 |
| 12.1100 | 3.6716 | 26.41 | 173 |
| 12.3950 | 3.5884 | 58.47 | 383 |
| 12.6400 | 3.5200 | 100.00 | 655 |
| 12.9450 | 3.4384 | 30.84 | 202 |
| 13.1550 | 3.3845 | 56.64 | 371 |
| 13.8350 | 3.2211 | 14.66 | 96 |
| 14.0550 | 3.1717 | 24.12 | 158 |
| 14.3550 | 3.1068 | 36.34 | 238 |
| 14.6700 | 3.0415 | 42.90 | 281 |
| 15.1300 | 2.9511 | 42.90 | 281 |
| 15.7500 | 2.8377 | 18.47 | 121 |
| 15.9400 | 2.8047 | 18.17 | 119 |
| 16.2500 | 2.7526 | 15.42 | 101 |
| 16.7550 | 2.6719 | 17.71 | 116 |
| 16.8500 | 2.6573 | 19.39 | 127 |
| 17.2250 | 2.6011 | 18.17 | |
| 17.7300 | 2.5293 | 15.73 | 103 |
| 18.4200 | 2.4377 | 10.23 | 67 |
| 18.5750 | 2.4180 | 10.23 | 67 |
| 19.2650 | 2.3345 | 15.88 | 104 |
| 19.5800 | 2.2984 | 10.84 | 71 |
| 19.8000 | 2.2739 | 11.91 | 78 |
| 20.1150 | 2.2397 | 13.28 | 87 |
| 20.6500 | 2.1841 | 14.81 | 97 |
| 21.1500 | 2.1348 | 11.30 | 74 |
| 21.2750 | 2.1228 | 10.08 | 66 |
| 21.8100 | 2.0732 | 7.94 | 52 |
| 22.2500 | 2.0342 | 9.16 | 60 |
| 22.6250 | 2.0022 | 9.62 | 63 |
| 22.9100 | 1.9786 | 9.01 | 59 |
| Longueur d'onde : 1.54051 Å | | | |

Ces cristaux sont filtrés puis lavés avec un mélange éthanol/acétone/eau (40/40/20). Le lavage reconvertit les cristaux de fluorescéine jaune en cristaux de fluorescéine rouge dont la pureté est plus élevée.

Pureté en CLHP : 99,8%

**EXEMPLE 2: Préparation de la 4',5'-diméthylfluorescéine de pureté élevée.**

**Synthèse de la 4' ,5'-diméthylfluorescéine**

Un mélange comprenant 62 g de 2-méthylrésorcinol, 30 g d'anhydride phtalique, 2g d'hydrogénosulfate de potassium et 500 ml de laurate d'éthyle est porté à 200°C pendant 3 heures. Après refroidissement, le produit brut est filtré et lavé à l'acétone puis séché. Le produit obtenu est une poudre orange foncé.

Masse obtenue = 49,7 g (69%)

Ces cristaux sont analysés par radiocristallographie de la même façon que dans l'exemple 1. On obtient le radiocristallographe de la figure 3, dont les pics sont identifiés ci-après.

| Thêta (degrés) | Distances Å | % Intensités | Nbre de coups |
|---|---|---|---|
| 3,5700 | 12.3700 | 7.38 | 81 |
| 5.0290 | 8.7938 | 45.49 | 499 |
| 6.6650 | 6.6365 | 62.26 | 683 |
| 6.7400 | 6.5630 | 60.07 | 659 |
| 7.1300 | 6.2057 | 87.24 | 957 |
| 7.4550 | 5.9366 | 20.97 | 230 |
| 8.3700 | 5.2915 | 6.56 | 72 |
| 8.9300 | 4.9621 | 30.63 | 336 |
| 9.0950 | 4.8728 | 10.12 | 111 |
| 9.3300 | 4.7511 | 21.24 | 233 |
| 9.4550 | 4.6889 | 19.05 | 209 |
| 10.0350 | 4.4204 | 12.12 | 133 |
| 10.3250 | 4.2975 | 18.87 | 207 |
| 10.6000 | 4.1873 | 11.49 | 126 |
| 10.8300 | 4.0994 | 39.65 | 435 |
| 11.2900 | 3.9344 | 12.49 | 137 |
| 11.4500 | 3.8801 | 10.85 | 119 |
| 11.7550 | 3.7808 | 28.17 | 309 |
| 11.9050 | 3.7339 | 52.42 | 575 |
| 12.2550 | 3.6288 | 79.95 | 877 |
| 12.7050 | 3.5023 | 8.11 | 89 |
| 13.2400 | 3.3631 | 21.15 | 232 |
| 13.4200 | 3.3188 | 100.00 | 1097 |
| 13.7100 | 3.2499 | 10.85 | 119 |
| 14.5250 | 3.0712 | 8.84 | 97 |
| 14.8350 | 3.0084 | 23.25 | 255 |
| 15.0550 | 2.9654 | 17.59 | 193 |
| 15.1550 | 2.9463 | 15.13 | 166 |
| 15.3650 | 2.9070 | 25.80 | 283 |
| 15.7050 | 2.8456 | 20.51 | 225 |
| 16.1900 | 2.7625 | 14.13 | 155 |
| 16.7550 | 2.6719 | 9.02 | 99 |
| 17.5400 | 2.5558 | 7.47 | 82 |
| 17.8850 | 2.5081 | 8.75 | 96 |
| 18.1050 | 2.4786 | 6.75 | 74 |
| 18.4900 | 2.4288 | 19.51 | 214 |
| 18.7950 | 2.3907 | 6.11 | 67 |
| 18.8900 | 2.3791 | 6.84 | 75 |

(suite)

| Thêta (degrés) | Distances Å | % Intensités | Nbre de coups |
|---|---|---|---|
| 19.6800 | 2.2872 | 15.50 | 170 |
| 20.2100 | 2.2296 | 7.38 | 81 |
| 20.3950 | 2.2103 | 9.66 | 106 |
| 20.6800 | 2.1811 | 6.84 | 75 |
| 20.9950 | 2.1498 | 5.38 | 59 |
| 21.4650 | 2.1049 | 7.11 | 78 |
| 21.6500 | 2.0878 | 8.84 | 97 |
| 21.9350 | 2.0620 | 9.21 | 101 |
| 22.1250 | 2.0451 | 9.02 | 99 |
| 22.5300 | 2.0102 | 5.47 | 60 |
| 22.7850 | 1.9889 | 5.93 | 65 |
| Longueur d'onde : 1.54051 Å | | | |

**Purification de la 4' ,5'-diméthylfluorescéine**

40 g de 4' ,5' -diméthylfluorescéine brute sont ajoutés dans 800 ml de mélange éthanol/acétone. A ce mélange est ajouté de l'acide sulfurique concentré jusqu'à conversion complète de cristaux de couleur orange foncé en cristaux de couleur jaune.

Les cristaux jaunes obtenus sont analysés par radiocristallographie comme précédemment. On obtient le radiocristallographe de la figure 4, dont les pics sont identifiés ci-après.

| Thêta (degrés) | Distances (Å) | % Intensités | Nbre de coups |
|---|---|---|---|
| 3.7050 | 11.9199 | 22.22 | 172 |
| 4.6150 | 9.5732 | 45.61 | 353 |
| 4.9300 | 8.9629 | 46.77 | 362 |
| 6.3300 | 6.9861 | 8.53 | 66 |
| 6.6150 | 6.6864 | 6.20 | 48 |
| 7.3000 | 6.0619 | 19.51 | 151 |
| 7.4500 | 5.9405 | 32.56 | 252 |
| 7.6500 | 5.7861 | 26.61 | 206 |
| 8.1250 | 5.4499 | 64.21 | 497 |
| 8.6300 | 5.1332 | 22.48 | 174 |
| 9.5950 | 4.6211 | 6.59 | 51 |
| 10.4450 | 4.2487 | 41.34 | 320 |
| 10.8500 | 4.0919 | 10.21 | 79 |
| 11.3550 | 3.9122 | 14.60 | 113 |
| 11.7000 | 3.7983 | 9.17 | 71 |
| 11.7950 | 3.7682 | 9.30 | 72 |
| 12.2250 | 3.6375 | 23.13 | 179 |
| 12.4500 | 3.5728 | 58.79 | 455 |
| 12.7650 | 3.4861 | 19.90 | 154 |
| 12.8950 | 3.4515 | 18.60 | 144 |
| 13.1000 | 3.3984 | 100.00 | 774 |
| 13.6350 | 3.2675 | 67.44 | 522 |
| 14.1400 | 3.1530 | 30.75 | 238 |
| 14.5250 | 3.0712 | 14.08 | 109 |
| 14.8400 | 3.0074 | 22.74 | 176 |
| 15.1750 | 2.9425 | 19.38 | 150 |
| 15.3500 | 2.9098 | 30.23 | 234 |
| 15.695C | 2.8474 | 20.93 | 162 |

(suite)

| Thêta (degrés) | Distances (Å) | % Intensités | Nbre de coups |
|---|---|---|---|
| 16.0950 | 2.7784 | 7.24 | 56 |
| 16.2850 | 2.7468 | 7.49 | 58 |
| 16.5950 | 2.6969 | 8.01 | 62 |
| 16.9100 | 2.6481 | 14.08 | 109 |
| 17.3200 | 2.5873 | 8.66 | 67 |
| 17.5400 | 2.5558 | 7.88 | 61 |
| 17.9150 | 2.5040 | 13.31 | 103 |
| 18.3550 | 2.4460 | 9.69 | 75 |
| 18.4800 | 2.4300 | 10.59 | 82 |
| 18.8600 | 2.3828 | 15.37 | 119 |
| 19.2350 | 2.3380 | 9.43 | 73 |
| 19.4850 | 2.3092 | 7.75 | 60 |
| 20.2100 | 2.2296 | 14.99 | 116 |
| 20.7100 | 2.1781 | 7.11 | 55 |
| 20.8350 | 2.1656 | 6.59 | 51 |
| 21.0850 | 2.1411 | 6.33 | 49 |
| 21.5250 | 2.0993 | 12.92 | 100 |
| 21.6500 | 2.0878 | 8.79 | 68 |
| 21.8400 | 2.0705 | 6.98 | 54 |
| 22.0900 | 2.0482 | 10.34 | 80 |
| 226550 | 1.9997 | 7.11 | 55 |
| Longueur d'onde : 1.54051 Å | | | |

Après filtration et recristallisation dans un mélange acétone/eau ou lavage dans éthanol/acétone/eau, les cristaux de couleur jaune se reconvertissent en cristaux de couleur orange foncé.

**EXEMPLE 3: Préparation de la 4',5'-dihydroxyfluorescéine**

**Synthèse de la 4' ,5'-dihydroxyfluorescéine**

Un mélange comprenant 63 g de pyrogallol, 30 g d'anhydride phtalique, 2g d'hydrogénosulfate de potassium et 500 ml de myristate d'éthyle est porté à 200°C pendant 3 heures. Après refroidissement, le produit brut est filtré et lavé à l'acétone puis séché. Le produit obtenu est une poudre marron-gris ou anthracite.

```
Masse obtenue = 43,5 g (59,7%)
```

Masse obtenue = 43,5 g (59,7%)

Les cristaux obtenus sont analysés par radiocristallographie comme précédemment. On obtient le radiocristallographe de la figure 5, dont les pics sont identifiés ci-après.

| Thêta (degrés) | Distances (Å) | % Intensités | Nbre de coups |
|---|---|---|---|
| 3.6450 | 12.1158 | 73.08 | 980 |
| 5.9200 | 7.4681 | 8.13 | 109 |
| 6.7200 | 6.5824 | 13.35 | 179 |
| 7.3350 | 6.0331 | 10.29 | 138 |
| 8.5050 | 5.2081 | 11.78 | 158 |
| 9.2300 | 4.8021 | 15.59 | 209 |
| 9.7400 | 4.5529 | 18.20 | 244 |
| 10.3400 | 4.2914 | 21.40 | 287 |
| 10.9150 | 4.0678 | 6.71 | 90 |

(suite)

| Thêta (degrés) | Distances (Å) | % Intensités | Nbre de coups |
|---|---|---|---|
| 11.2600 | 3.9447 | 8.80 | 118 |
| 12.2550 | 3.6288 | 18.64 | 250 |
| 12.5550 | 3.5434 | 12.75 | 171 |
| 13.2000 | 3.3731 | 11.86 | 159 |
| 13.7200 | 3.2476 | 100.00 | 1341 |
| 14.7150 | 3.0324 | 17,75 | 238 |
| 14.8600 | 3.0034 | 13.57 | 182 |
| 16.1600 | 2.7675 | 6.79 | 91 |
| 17.2550 | 2.5967 | 7.83 | 105 |
| 18.3550 | 2.4460 | 6.86 | 92 |
| Longueur d'onde : 1.54051 Å | | | |

**Purification de la 4' ,5'-dihydroxyfluorescéine**

40 g de 4' ,5' -dihydroxyfluorescéine brute sont ajoutés dans 800 ml de mélange éthanol/acétone. A ce mélange est ajouté de l'acide sulfurique concentré jusqu'à conversion complète de cristaux de couleur marron-gris ou anthracite en cristaux de couleur marron-rouge ou acajou.

Les cristaux obtenus sont analysés par radiocristallographie comme précédemment. On obtient le radiocristallographe de la figure 6, dont les pics sont identifiés ci-après.

| Thêta (degrés) | Distances (Å) | % Intensités | Nbre de coups |
|---|---|---|---|
| 3.2500 | 13.5865 | 90.53 | 526 |
| 3.5350 | 12.4923 | 18.76 | 109 |
| 3.7850 | 11.6683 | 11.70 | 68 |
| 3.9450 | 11.1958 | 13.25 | 77 |
| 4.1650 | 10.6053 | 15.15 | 88 |
| 4.5400 | 9.7310 | 10.33 | 60 |
| 4.6350 | 9.5319 | 10.15 | 59 |
| 6.3950 | 6.9154 | 9.29 | 54 |
| 6.5500 | 6.7525 | 11.53 | 67 |
| 6.8200 | 6.4863 | 27.71 | 161 |
| 7.1150 | 6.2187 | 10.50 | 61 |
| 7.4800 | 5.9168 | 21.51 | 125 |
| 7.8700 | 5.6253 | 12.91 | 75 |
| 8.2200 | 5.3874 | 27.88 | 162 |
| 8.7800 | 5.0462 | 41.48 | 241 |
| 9.2200 | 4.8073 | 10.15 | 59 |
| 9.5350 | 4.6499 | 11.70 | 68 |
| 9.7950 | 4.5276 | 42.17 | 245 |
| 10.1000 | 4.3923 | 17.21 | 100 |
| 10.5400 | 4.2108 | 16.70 | 97 |
| 10.8850 | 4.0789 | 16.52 | 96 |
| 11.6500 | 3.8144 | 25.13 | 146 |
| 11.8900 | 3.7385 | 13.60 | 79 |
| 12.0450 | 3.6911 | 13.08 | 76 |
| 12.3900 | 3.5898 | 16.52 | 96 |
| 12.5800 | 3.5365 | 14.80 | 86 |
| 12.8750 | 3.4568 | 27.37 | 159 |
| 12.9950 | 3.4254 | 25.82 | 150 |
| 13.2900 | 3.3507 | 67.64 | 393 |

(suite)

| Thêta (degrés) | Distances (Å) | % Intensités | Nbre de coups |
|---|---|---|---|
| 13.5750 | 3.2816 | 27.02 | 157 |
| 13.7150 | 3.2488 | 27.02 | 157 |
| 14.1100 | 3.1596 | 100.00 | 581 |
| 14.6200 | 3.0516 | 15.66 | 91 |
| 14.7750 | 3.0203 | 18.24 | 106 |
| 15.0400 | 2.9683 | 25.13 | 146 |
| 15.3050 | 2.9181 | 24.78 | 144 |
| 15.6850 | 2.8491 | 16.35 | 95 |
| 15.9400 | 2.8047 | 11.02 | 64 |
| 16.2500 | 2.7526 | 10.67 | 62 |
| 16.5950 | 2.6969 | 12.22 | 71 |
| 17.1650 | 2.6099 | 9.81 | 57 |
| 17.5700 | 2.5516 | 8.78 | 51 |
| 17.7600 | 2.5252 | 9.81 | 57 |
| 18.0750 | 2.4826 | 11.36 | 66 |
| 18.5150 | 2.4256 | 8.43 | 49 |
| 18.7650 | 2.3944 | 13.08 | 76 |
| 19.0450 | 2.3605 | 11.36 | 66 |
| 20.1950 | 2.2312 | 50,09 | 291 |
| 20.6150 | 2.1877 | 9.12 | 53 |
| 21.0550 | 2.1440 | 12.56 | 73 |
| 21.4650 | 2.1049 | 9.29 | 54 |
| 21.6850 | 2.0846 | 9.98 | 58 |
| 22.0600 | 2.0509 | 9.81 | 57 |
| Longueur d'onde : 1.54051 Å | | | |

Après filtration et lavage dans l'eau, les cristaux de couleur marron-rouge ou acajou se reconvertissent en cristaux de couleur marron-gris ou anthracite.

## Revendications

1. Procédé de préparation des phtaléines, débarrassées de leurs impuretés résiduelles, présentant la formule générale (I) :

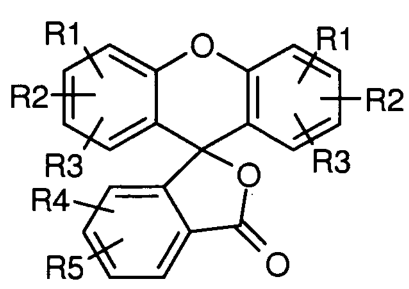

(I)

dans laquelle R1, R2, R3, R4, R5 qui sont identiques ou différents les uns des autres, sont choisis dans le groupe comprenant les radicaux ou groupements suivants : hydrogène, hydroxyle, halogène, acétyle, amino,

phosphate, nitro, sulfonate, carboxy, alkylcarboxy de 2 à 30 atomes de carbone, alkyle ayant de 1 à 30 atomes de carbone, cycloalkyle ayant de 3 à 12 atomes de carbone, alkyloxy ayant de 1 à 30 atomes de carbone, halogénoalkyle ayant de 1 à 30 atomes de carbone, hydroxyalkyle ayant de 1 à 30 atomes de carbone, alkylester ayant de 2 à 40 atomes de carbone, nitroalkyle ayant de 1 à 30 atomes de carbone, carboxyalkyle ayant de 2 à 30 atomes de carbone, aminoalkyle ayant de 1 à 30 atomes de carbone, sulfoalkyle ayant de 1 à 30 atomes de carbone, aryle, aryloxy, aryl-alkyle, halogénoaryle, arylester, succinimidylester, isothiocyanate, maléimide, iodoacétamide, halogénoacétamide, chlorosulfonique, les bases puriques ou pyrimidiques, les oses, de préférence les hexoses ou les pentoses, les oligosides et les polyosides, les polypeptides, les protéines et les phospholipides,
R3 et R5 ne représentant pas chacun l'hydrogène lorsque R1 représente un groupe $-CH_2-CH_2-COOH$, R2 représente un groupe hydroxy et R4 représente un groupe -COOH,
**caractérisé par le fait que** l'on condense un dérivé de l'anhydride phtalique de formule (II) avec un composé phénolique ou naphtolique de formule (III)

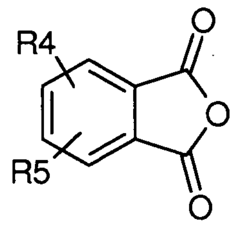

(II)

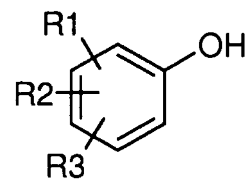

(III)

dans lesquelles R1, R2, R3, R4, R5 ont les mêmes significations que ci-dessus,
la condensation étant effectuée dans un solvant constitué par un ester d'acide organique.

**2.** Procédé selon la revendication 1, dans lequel le composé de formule (III) est choisi dans le groupe comprenant le résorcinol, l'orcinol, le naphtol, le pyrogallol, l'alkylaminophénol et l'arylaminophénol.

**3.** Procédé selon l'une des revendications 1 et 2, dans lequel le solvant est un ester d'acide organique de formule (IV)

$$R_6\text{-}COOR_7 \qquad (IV)$$

dans laquelle $R_6$ est choisi dans le groupe comprenant les radicaux ou groupements suivants : hydrogène, alkyle ayant de 1 à 30 atomes de carbone, cycloalkyle ayant de 3 à 12 atomes de carbone, halogénoalkyle ayant de 1 à 30 atomes de carbone, hydroxyalkyle ayant de 1 à 30 atomes de carbone, nitroalkyle ayant de 1 à 30 atomes de carbone, aryle, aryloxy, alkyl-aryle, aryl-alkyle, aryl-alkyle substitué, halogénoaryle, arylester, alkylester ayant de 2 à 40 atomes de carbone, alkyloxy ayant de 1 à 30 atomes de carbone, $R_7$ représentant un groupe alkyle ayant de 1 à 30 atomes de carbone, cycloalkyle ayant de 3 à 12 atomes de carbone, halogénoalkyle ayant de 1 à 30 atomes de carbone, hydroxyalkyle ayant de 1 à 30 atomes de carbone, nitroalkyle ayant de 1 à 30 atomes de carbone, aryle, aryloxy, alkyl-aryle, aryl-alkyle, aryl-alkyle substitué, halogénoaryle, arylester, alkylester ayant de 2 à 40 atomes de carbone, alkyloxy ayant de 1 à 30 atomes de carbone.

**4.** Procédé selon l'une des revendications 1 à 3, **caractérisé par le fait que** l'ester d'acide organique est choisi dans le groupe comprenant le benzoate, l'heptanoate, l'octanoate, le laurate, le myristate ou le palmitate de méthyle, d'éthyle, de propyle ou de butyle, et leurs mélanges.

**5.** Procédé selon l'une des revendications 1 à 4, **caractérisé par le fait que** la réaction de condensation est conduite entre 150˚C et 250˚C, éventuellement sous pression.

**6.** Procédé selon l'une des revendications 1 à 5, **caractérisé par le fait qu'**il est conduit en présence d'un catalyseur choisi dans le groupe comprenant notamment les acides de Lewis, tels que $ZnCl_2$ ou $AlCl_3$, les acides de Bronsted tels que $H_2SO_4$ ou l'acide polyphosphorique, de préférence un hydrogénosulfate alcalin et plus préférentiellement l'hydrogénosulfate de potassium ($KHSO_4$) ou l'hydrogénosulfate de sodium ($NaHSO_4$).

**7.** Procédé d'acidification du produit résultant de la condensation d'un dérivé de l'anhydride phtalique de formule (II) avec un composé phénolique ou naphtolique de formule (III), les formules (II) et (III) étant celles de la revendication 1, **caractérisé par le fait qu'**il est conduit en milieu organique anhydre, par addition d'un acide fort ou un de ses précurseurs choisi dans le groupe comprenant l'acide sulfurique, l'acide chlorhydrique, l'acide bromhydrique, l'acide fluorhydrique, l'acide iodhydrique, l'acide polyphosphorique, le pyrophosphate ($P_2O_5$) et leurs mélanges, l'acidification étant menée jusqu'à conversion des cristaux de phtaléine résultant de la condensation en cristaux de phtaléine de structure différente.

**8.** Procédé selon la revendication 7, **caractérisé par le fait que** le produit de condensation est le produit obtenu par le procédé selon l'une quelconque des revendications 1 à 6.

**9.** Procédé selon la revendication 7 ou 8, **caractérisé par le fait qu'**il comporte une étape de lavage du produit obtenu après acidification, ladite étape de lavage étant effectuée à l'aide d'une solution de lavage choisie dans le groupe comprenant l'eau, les alcools, les cétones, les éthers et les solvants halogénés, purs ou en mélange jusqu'à reconversion des cristaux dans la structure qu'ils avaient avant la réaction d'acidification.

**10.** Procédé de préparation d'une fluorescéine présentant une pureté telle que sa teneur en chacun des sous-produits de la réaction est inférieure ou égale à 0,2% et de préférence inférieure ou égale à 0,1%, la somme des teneurs en chacun de ces sous-produits étant inférieure ou égale à 0,5%, ledit procédé comprenant les étapes successives suivantes:

- condensation de l'anhydride phtalique avec le résorcinol, dans un solvant constitué par un ester d'acide organique aliphatique ou aromatique, de préférence le benzoate ou le palmitate d'éthyle ou de méthyle, en présence d'un catalyseur choisi dans le groupe comprenant notamment les acides de Lewis ou les acides de Bronsted, et de préférence un hydrogénosulfate alcalin,
- mise en suspension des cristaux de couleur rouge obtenus à l'étape précédente dans un solvant anhydre choisi dans le groupe comprenant les alcools tel que l'éthanol absolu, les cétones telles que l'acétone, les éthers, les solvants halogénés, ou leurs mélanges,
- acidification de la suspension ainsi obtenue par addition d'un acide fort ou un de ses précurseurs choisi dans le groupe comprenant notamment l'acide sulfurique, l'acide chlorhydrique, l'acide bromhydrique, l'acide fluorhydrique, l'acide iodhydrique, l'acide polyphosphorique, le pyrophosphate ($P_2O_5$), et leurs mélanges, jusqu'à conversion des cristaux de couleur rouge en cristaux de couleur jaune présentant le spectre de radiocristallographie de la figure 2,
- lavage des cristaux obtenus à l'aide d'une solution de lavage choisie dans le groupe comprenant l'eau, les alcools, les cétones, les éthers et les solvants halogénés, purs ou en mélange, ce lavage étant poursuivi jusqu'à la reconversion des cristaux de couleur jaune en cristaux de couleur rouge.

**11.** Procédé selon l'une des revendications 7 à 10, **caractérisé par le fait que** l'acidification est réalisée par barbotage d'acide chlorhydrique gazeux dans la suspension de phtaléine ou par l'action sur cette phtaléine d'acide chlorhydrique en solution dans le solvant organique anhydre, de préférence un alcool, une cétone, un éther, un solvant halogéné utilisés isolément ou en mélange, plus préférentiellement encore, l'isopropanol, l'éthanol absolu ou l'acétone, purs ou en mélange.

**12.** Procédé selon l'une des revendications 1 à 11, **caractérisé par le fait que** le catalyseur utilisé pour la réaction de condensation est constitué par l'hydrogénosulfate d'un métal alcalin, de préférence de l'hydrogénosulfate de potassium ou de sodium.

**13.** Cristaux de fluorescéine de couleur jaune présentant le spectre de radiocristallographie dont les pics sont identifiés ci-après :

| Thêta (degrés) | Distances (Å) | % Intensités | Nbre de coups |
|---|---|---|---|
| 3.3500 | 13.1813 | 8.55 | 56 |
| 4.0050 | 11.0283 | 47.18 | 309 |
| 4.8550 | 9.1010 | 12.98 | 85 |
| 5.2650 | 8.3940 | 9.01 | 59 |
| 5.4500 | 8.1099 | 11.45 | 75 |

(suite)

| Thêta (degrés) | Distances (Å) | % Intensités | Nbre de coups |
|---|---|---|---|
| 5.7900 | 7.6352 | 52.98 | 347 |
| 6.3000 | 7.0193 | 16.95 | 111 |
| 6.7700 | 6.5340 | 17.86 | 117 |
| 7.4900 | 5.9090 | 17.56 | 115 |
| 8.0300 | 5.5140 | 69.77 | 457 |
| 8.4250 | 5.2572 | 33.13 | 217 |
| 8.7800 | 5.0462 | 8.40 | 55 |
| 9.1550 | 4.8411 | 15.88 | 104 |
| 9.3400 | 4.7461 | 42.44 | 278 |
| 9.6550 | 4.5926 | 34.66 | 227 |
| 9.8800 | 4.4891 | 24.43 | 160 |
| 10.1950 | 4.3518 | 57.71 | 378 |
| 10.5800 | 4.1951 | 58.02 | 380 |
| 10.7350 | 4.1352 | 38.17 | 250 |
| 11.1650 | 3.9779 | 28.85 | 189 |
| 11.4800 | 3.8701 | 30.23 | 198 |
| 11.7200 | 3.7919 | 59.85 | 392 |
| 12.1100 | 3.6716 | 26.41 | 173 |
| 12.3950 | 3.5884 | 58.47 | 383 |
| 12.6400 | 3.5200 | 100.00 | 655 |
| 12.9450 | 3.4384 | 30.84 | 202 |
| 13.1550 | 3.3845 | 56.64 | 371 |
| 13.8350 | 3.2211 | 14.66 | 96 |
| 14.0550 | 3.1717 | 24.12 | 158 |
| 14.3550 | 3.1068 | 36.34 | 238 |
| 14.6700 | 3.0415 | 42.90 | 281 |
| 15.1300 | 2.9511 | 42.90 | 281 |
| 15.7500 | 2.8377 | 18.47 | 121 |
| 15.9400 | 2.8047 | 18.17 | 119 |
| 16.2500 | 2.7526 | 15.42 | 101 |
| 16,7550 | 2.6719 | 17.71 | 116 |
| 16.8500 | 2.6573 | 19.39 | 127 |
| 17.2250 | 2.6011 | 18.17 | 119 |
| 17.7300 | 2.5293 | 15.73 | 103 |
| 18.4200 | 2.4377 | 10.23 | 67 |
| 18.5750 | 2.4180 | 10.23 | 67 |
| 19.2650 | 2.3345 | 15.88 | 104 |
| 19.5800 | 2.2984 | 10.84 | 71 |
| 19.8000 | 2.2739 | 11.91 | 78 |
| 20.1150 | 2.2397 | 13.28 | 87 |
| 20.6500 | 2.1841 | 14.81 | 97 |
| 21.1500 | 2.1348 | 11.30 | 74 |
| 21.2750 | 2.1228 | 10.08 | 66 |
| 21.8100 | 2.0732 | 7.94 | 52 |
| 22.2500 | 2.0342 | 9.16 | 60 |
| 22.6250 | 2.0022 | 9.62 | 63 |
| 22.9100 | 1.9786 | 9.01 | 59 |

Longueur d'onde : 1.54051 Å.

**14.** Cristaux de fluorescéine susceptibles d'être obtenus par un procédé selon l'une quelconque des revendications 7

à 11.

**15.** Utilisation des cristaux de fluorescéine selon la revendication 14 dans des applications pharmaceutiques en diagnostic notamment en imagerie médicale ou dans le domaine des applications en biotechnologie.

## Claims

**1.** A method for preparing phthaleins, wherein the residual impurities have been removed, having the general formula (I):

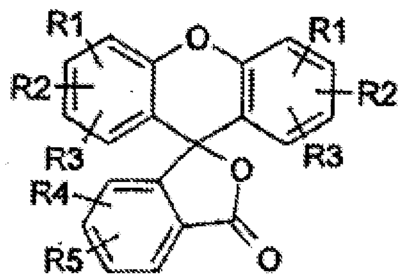

(I)

wherein

R1, R2, R3, R4 and R5, which are identical to or different from one another, are selected from the group comprising the following radicals or moieties: hydrogen, hydroxyl, halogen, acetyl, amino, phosphate, nitro, sulfonate, carboxy, alkylcarboxy having from 2 to 30 carbon atoms, alkyl having from 1 to 30 carbon atoms, cycloalkyl having from 3 to 12 carbon atoms, alkyloxy having from 1 to 30 carbon atoms, haloalkyl having from 1 to 30 carbon atoms, hydroxyalkyl having from 1 to 30 carbon atoms, alkyl ester having from 2 to 40 carbon atoms, nitroalkyl having from 1 to 30 carbon atoms, carboxyalkyl having from 2 to 30 carbon atoms, aminoalkyl having from 1 to 30 carbon atoms, sulfoalkyl having from 1 to 30 carbon atoms, aryl, aryloxy, arylalkyl, haloaryl, aryl ester, succinimidyl ester, isothiocyanate, maleimide, iodoacetamide, haloacetamide, chlorosulfonic, purine or pyrimidine bases, monosaccharides, preferably hexoses or pentoses, oligosides and polyosides, polypeptides, proteins and phospholipids,
R3 and R5 are not hydrogen when R1 is a group $-CH_2-CH_2-COOH$, R2 is a hydroxyl group and R4 is a group $-COOH$,
wherein a phthalic anhydride derivative of formula (II) is condensed with a phenol or naphthol compound of formula (III)

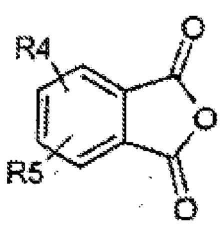

(II)

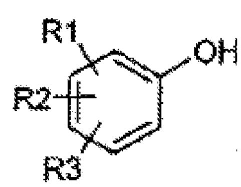

(III)

in which R1, R2, R3, R4 and R5 have the same meanings as above,
the condensation being carried out in a solvent consisting of an organic acid ester.

**2.** The method according to claim 1, wherein the compound of formula (III) is selected from the group comprising

resorcinol, orcinol, naphthol, pyrogallol, alkylaminophenol and arylaminophenol.

**3.** The method according to any one of claims 1-2, wherein the solvent is an organic acid ester of formula (IV)

$$R_6\text{-}COOR_7 \qquad (IV)$$

wherein $R_6$ is selected from the group comprising the following radicals and moieties: hydrogen, alkyl having from 1 to 30 carbon atoms, cycloalkyl having from 3 to 12 carbon atoms, haloalkyl having from 1 to 30 carbon atoms, hydroxyalkyl having from 1 to 30 carbon atoms, nitroalkyl having from 1 to 30 carbon atoms, aryl, aryloxy, alkylaryl, arylalkyl, substituted arylalkyl, haloaryl, aryl ester, alkyl ester having from 2 to 40 carbon atoms, and alkyloxy having from 1 to 30 carbon atoms, $R_7$ being an alkyl having from 1 to 30 carbon atoms, a cycloalkyl having from 3 to 12 carbon atoms, a haloalkyl having from 1 to 30 carbon atoms, a hydroxyalkyl having from 1 to 30 carbon atoms, a nitroalkyl having from 1 to 30 carbon atoms, an aryl, an aryloxy, an alkylaryl, an arylalkyl, a substituted arylalkyl, a haloaryl, an aryl ester, an alkyl ester having from 2 to 40 carbon atoms, or an alkyloxy having from 1 to 30 carbon atoms.

**4.** The method according to any one of claims 1-3, wherein the organic acid ester is selected from the group comprising methyl, ethyl, propyl or butyl benzoate, methyl, ethyl, propyl or butyl heptanoate, methyl, ethyl, propyl or butyl octanoate, methyl, ethyl, propyl or butyl laurate, methyl, ethyl, propyl or butyl myristate or methyl, ethyl, propyl or butyl palmitate, and mixtures thereof.

**5.** The method according to any one of claims 1-4, wherein the condensation reaction is carried out between 150˚C and 250˚C, optionally under pressure.

**6.** The method according to any one of claims 1-5, wherein the reaction is carried out in the presence of a catalyst selected from the group comprising Lewis acids, such as $ZnCl_2$ or $AlCl_3$, Brönsted acids such as $H_2SO_4$ or polyphosphoric acid, preferably an alkali metal hydrogen sulphate, more preferably potassium hydrogen sulfate ($KHSO_4$) or sodium hydrogen sulfate ($NaHSO_4$).

**7.** A method for acidifying the product resulting from the condensation of a phthalic anhydride derivative of formula (II) with a phenol or naphthol compound of formula (III), the formulae (II) and (III) being those of claim 1, wherein the reaction is carried out in an anhydrous organic medium, by the addition of a strong acid or one of its precursors selected from the group comprising sulfuric acid, hydrochloric acid, hydrobromic acid, hydrofluoric acid, hydriodic acid, polyphosphoric acid, pyrophosphate ($P_2O_5$), and mixtures thereof, the acidification being carried out until the phthalein crystals resulting from the condensation are converted to phthalein crystals having a different structure.

**8.** The method according to claim 7, wherein the condensation product is the product obtained by the method as defined in any one of claims 1-6.

**9.** The method according to claim 7 or 8, wherein it comprises a step of washing the product obtained after acidification, said washing step being carried out with a washing solution selected from the group comprising water, alcohols, ketones, ethers and halogenated solvents, pure or as a mixture, until the crystals are reconverted to the structure that they had before the acidification reaction.

**10.** A method for preparing a fluorescein having a purity such that its content of each of the by-products of the reaction is less than or equal to 0.2%, preferably less than or equal to 0.1%, the sum of the contents of each of these by-products being less than or equal to 0.5%, said method comprising the following successive steps:

- condensing phthalic anhydride with resorcinol, in a solvent consisting of an ester of an aliphatic or aromatic organic acid, preferably ethyl benzoate or ethyl palmitate, in the presence of a catalyst selected from the group comprising Lewis acids or Brönsted acids, preferably an alkali metal hydrogen sulphate,
- suspending the red-colored crystals obtained in the preceding step in an anhydrous solvent selected from the group comprising alcohols such as absolute ethanol, ketones such as acetone, ethers, halogenated solvents, or mixtures thereof,
- acidifying the suspension thus obtained by the addition of a strong acid or one of its precursors, selected from the group comprising sulfuric acid, hydrochloric acid, hydrobromic acid, hydrofluoric acid, hydriodic acid, polyphosphoric acid, pyrophosphate ($P_2O_5$), and mixtures thereof, until the red-colored crystals are converted to yellow-colored crystals exhibiting the X-ray diffraction analysis of figure 2,
- washing the crystals obtained with a washing solution selected from the group comprising water, alcohols,

ketones, ethers and halogenated solvents, pure or as a mixture, this washing being continued until the yellow-colored crystals are reconverted to red-colored crystals.

**11.** The method according to any one of claims 7-10, wherein the acidification is carried out by sparging gaseous hydrochloric acid into the phthalein suspension or by the action, on this phthalein, of hydrochloric acid in solution in the anhydrous organic solvent, preferably an alcohol, a ketone, an ether or a halogenated solvent, used alone or as a mixture, even more preferably isopropanol, absolute ethanol or acetone, pure or as a mixture.

**12.** The method according to any one of claims 7-11, wherein the catalyst used for the condensation reaction consists of an alkali metal hydrogen sulphate, preferably potassium hydrogen sulfate or sodium hydrogen sulfate.

**13.** A yellow-colored fluorescein crystal having the radiocrystallography spectra which has the peaks identified hereafter:

| Theta (degrees) | Distances (Å) | % Intensities | Number of counts |
|---|---|---|---|
| 3.3500 | 13.1813 | 8.55 | 56 |
| 4.0050 | 11.0283 | 47.18 | 309 |
| 4.8550 | 9.1010 | 12.98 | 85 |
| 5.2650 | 8.3940 | 9.01 | 59 |
| 5.4500 | 8.1099 | 11.45 | 75 |
| 5.7900 | 7.6352 | 52.98 | 347 |
| 6.3000 | 7.0193 | 16.95 | 111 |
| 6.7700 | 6.5340 | 17.86 | 117 |
| 7.4900 | 5.9090 | 17.56 | 115 |
| 8.0300 | 5.5140 | 69.77 | 457 |
| 8.4250 | 5.2572 | 33.13 | 217 |
| 8.7800 | 5.0462 | 8.40 | 55 |
| 9.1550 | 4.8411 | 15.88 | 104 |
| 9.3400 | 4.7461 | 42.44 | 278 |
| 9.6550 | 4.5926 | 34.66 | 227 |
| 9.8800 | 4.4891 | 24.43 | 160 |
| 10.1950 | 4.3518 | 57.71 | 378 |
| 10.5800 | 4.1951 | 58.02 | 380 |
| 10.7350 | 4.1352 | 38.17 | 250 |
| 11.1650 | 3.9779 | 28.85 | 189 |
| 11.4800 | 3.8701 | 30.23 | 198 |
| 11.7200 | 3.7919 | 59.85 | 392 |
| 12.1100 | 3.6716 | 26.41 | 173 |
| 12.3950 | 3.5884 | 58.47 | 383 |
| 12.6400 | 3.5200 | 100.00 | 655 |
| 12.9450 | 3.4384 | 30.84 | 202 |
| 13.1550 | 3.3845 | 56.64 | 371 |
| 13.8350 | 3.2211 | 14.66 | 96 |
| 14.0550 | 3.1717 | 24.12 | 158 |
| 14.3550 | 3.1068 | 36.34 | 238 |
| 14.6700 | 3.0415 | 42.90 | 281 |
| 15.1300 | 2.9511 | 42.90 | 281 |
| 15.7500 | 2.8377 | 18.47 | 121 |
| 15.9400 | 2.8047 | 18.17 | 119 |
| 16.2500 | 2.7526 | 15.42 | 101 |
| 16,7550 | 2.6719 | 17.71 | 116 |
| 16.8500 | 2.6573 | 19.39 | 127 |
| 17.2250 | 2.6011 | 18.17 | 119 |

(continued)

| Theta (degrees) | Distances (Å) | % Intensities | Number of counts |
|---|---|---|---|
| 17.7300 | 2.5293 | 15.73 | 103 |
| 18.4200 | 2.4377 | 10.23 | 67 |
| 18.5750 | 2.4180 | 10.23 | 67 |
| 19.2650 | 2.3345 | 15.88 | 104 |
| 19.5800 | 2.2984 | 10.84 | 71 |
| 19.8000 | 2.2739 | 11.91 | 78 |
| 20.1150 | 2.2397 | 13.28 | 87 |
| 20.6500 | 2.1841 | 14.81 | 97 |
| 21.1500 | 2.1348 | 11.30 | 74 |
| 21.2750 | 2.1228 | 10.08 | 66 |
| 21.8100 | 2.0732 | 7.94 | 52 |
| 22.2500 | 2.0342 | 9.16 | 60 |
| 22.6250 | 2.0022 | 9.62 | 63 |
| 22.9100 | 1.9786 | 9.01 | 59 |
| Wavelenght: 1.54051 Å. | | | |

**14.** A fluorescein crystal obtainable by the method according to any one of claims 7-11.

**15.** Use of a fluorescein crystal according to claim 14 in pharmaceutical applications in diagnostic, in particular in medical imaging, or in the domain of biotechnology applications.

**Patentansprüche**

**1.** Verfahren zur Herstellung von Phthaleinen, die von ihren Restverunreinigungen befreit sind, der allgemeinen Formel (I):

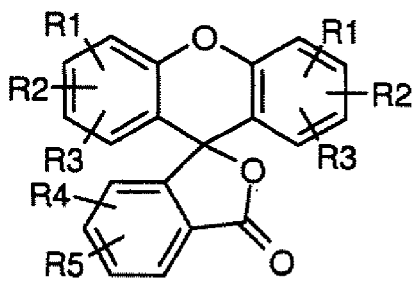

(I)

worin R1, R2, R3, R4 und R5 gleich oder voneinander verschieden sind und aus der Gruppe, die die folgenden Reste oder Gruppen enthält, ausgewählt sind: Wasserstoff, Hydroxyl, Halogen, Acetyl, Amino, Phosphat, Nitro, Sulfonat, Carboxyl, Alkylcarboxyl mit 2 bis 30 Kohlenstoffatomen, Alkyl mit 1 bis 30 Kohlenstoffatomen, Cycloalkyl mit 3 bis 12 Kohlenstoffatomen, Alkyloxy mit 1 bis 30 Kohlenstoffatomen, Halogenalkyl mit 1 bis 30 Kohlenstoffatomen, Hydroxyalkyl mit 1 bis 30 Kohlenstoffatomen, Alkylester mit 2 bis 40 Kohlenstoffatomen, Nitroalkyl mit 1 bis 30 Kohlenstoffatomen, Carboxyalkyl mit 2 bis 30 Kohlenstoffatomen, Aminoalkyl mit 1 bis 30 Kohlenstoffatomen, Sulfoalkyl mit 1 bis 30 Kohlenstoffatomen, Aryl, Aryloxy, Arylalkyl, Halogenaryl, Arylester, Succinimidylester, Isothiocyanat, Maleinimid, Iodacetamid, Halogenacetamid, Chlorsulfonyl, Purin- oder Pyrimidinbasen, Monosacchariden, vorzugsweise Hexosen oder Pentosen, Oligosiden und Polyosiden, Polypeptiden, Proteinen and Phospholipiden,

R3 und R5 nicht jeweils für Wasserstoff stehen, wenn R1 für eine Gruppe $-CH_2-CH_2-COOH$ steht, R2 für eine Hydroxylgruppe steht und R4 für eine Gruppe -COOH steht,
**dadurch gekennzeichnet, daß** man ein Phthalsäureanhydridderivat der Formel (II) mit einer Phenol- oder Naphtholverbindung der Formel (III)

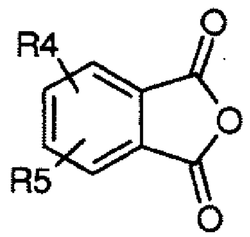

(II)

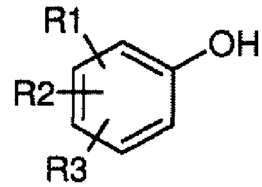

(III)

worin R1, R2, R3, R4 und R5 die gleichen Bedeutungen wie oben besitzen, kondensiert,
wobei die Kondensation in einem Lösungsmittel durchgeführt wird, das aus einem Ester einer organischen Säure besteht.

**2.** Verfahren nach Anspruch 1, wobei die Verbindung der Formel (III) aus der Gruppe enthaltend Resorcin, Orcin, Naphthol, Pyrogallol, Alkylaminophenol und Arylaminophenol auswählt wird.

**3.** Verfahren nach einem der Ansprüche 1 und 2, wobei das Lösungsmittel ein Ester einer organischen Säure der Formel (IV)

$R_6$-$COOR_7$ (IV)

worin $R_6$ aus der Gruppe, die die folgenden Reste oder Gruppen enthält, ausgewählt wird: Wasserstoff, Alkyl mit 1 bis 30 Kohlenstoffatomen, Cycloalkyl mit 3 bis 12 Kohlenstoffatomen, Halogenalkyl mit 1 bis 30 Kohlenstoffatomen, Hydroxyalkyl mit 1 bis 30 Kohlenstoffatomen, Nitroalkyl mit 1 bis 30 Kohlenstoffatomen, Aryl, Aryloxy, Alkylaryl, Arylalkyl, substituiertes Arylalkyl, Halogenaryl, Arylester, Alkylester mit 2 bis 40 Kohlenstoffatomen und Alkyloxy mit 1 bis 30 Kohlenstoffatomen, wobei $R_7$ für eine der folgenden Gruppen steht: Alkyl mit 1 bis 30 Kohlenstoffatomen, Cycloalkyl mit 3 bis 12 Kohlenstoffatomen, Halogenalkyl mit 1 bis 30 Kohlenstoffatomen, Hydroxyalkyl mit 1 bis 30 Kohlenstoffatomen, Nitroalkyl mit 1 bis 30 Kohlenstoffatomen, Aryl, Aryloxy, Alkylaryl, Arylalkyl, substituiertes Arylalkyl, Halogenaryl, Arylester, Alkylester mit 2 bis 40 Kohlenstoffatomen und Alkyloxy mit 1 bis 30 Kohlenstoffatomen, ist.

**4.** Verfahren nach einem der Ansprüche 1 bis 3, **dadurch gekennzeichnet, dass** der Ester einer organischen Säure aus der Gruppe enthaltend Benzoesäure-, Heptansäure-, Octansäure-, Laurinsäure-, Myristinsäure- oder Palmitinsäuremethylester, -ethylester, -propylester oder -butylester und Mischungen davon ausgewählt wird.

**5.** Verfahren nach einem der Ansprüche 1 bis 4, **dadurch gekennzeichnet, dass** die Kondensationsreaktion zwischen 150°C und 250°C durchgeführt wird, gegebenenfalls unter Druck.

**6.** Verfahren nach einem der Ansprüche 1 bis 5, **dadurch gekennzeichnet, dass** es in Gegenwart eines Katalysators ausgewählt aus der Gruppe, die insbesondere Lewis-Säuren, wie $ZnCl_2$ oder $AlCl_3$, Brönsted-Säuren, wie $H_2SO_4$ oder Polyphosphorsäure, vorzugsweise ein Alkalimetallhydrogensulfat und weiter bevorzugt Kaliumhydrogensulfat ($KHSO_4$) oder Natriumhydrogensulfat ($NaHSO_4$) enthält, durchgeführt wird.

**7.** Verfahren zum Ansäuern des Produkts der Kondensation eines Phthalsäureanhydridderivats der Formel (II) mit einer Phenol- oder Naphtholverbindung der Formel (III), wobei die Formeln (II) und (III) denjenigen von Anspruch 1 entsprechen, **dadurch gekennzeichnet, dass** es in einem wasserfreien organischen Medium durch Zugabe einer starken Säure oder eines Vorläufers davon ausgewählt aus der Gruppe enthaltend Schwefelsäure, Salzsäure, Bromwasserstoffsäure, Fluorwasserstoffsäure, Iodwasserstoffsäure, Polyphosphorsäure, Pyrophosphat ($P_2O_5$)

und Mischungen davon durchgeführt wird, wobei bis zur Umwandlung der aus der Kondensation erhaltenen Phthaleinkristalle in Phthaleinkristalle anderer Struktur angesäuert wird.

**8.** Verfahren nach Anspruch 7, **dadurch gekennzeichnet, dass** es sich bei dem Kondensationsprodukt um das durch das Verfahren nach einem der Ansprüche 1 bis 6 erhaltene Produkt handelt.

**9.** Verfahren nach Anspruch 7 oder 8, **dadurch gekennzeichnet, daß** es einen Schritt umfaßt, bei dem man das nach Ansäuern erhaltene Produkt wäscht, wobei der Waschschritt mit Hilfe einer Waschlösung ausgewählt aus der Gruppe enthaltend Wasser, Alkohole, Ketone, Ether und halogenierte Lösungsmittel in reiner Form oder als Mischung bis zur Rückumwandlung der Kristalle in die Struktur, die sie vor der Ansäuerungsreaktion hatten, durchgeführt wird.

**10.** Verfahren zur Herstellung eines Fluoresceins mit einer solchen Reinheit, dass sein Gehalt an jedem der Reaktionsnebenprodukte kleiner gleich 0,2% und vorzugsweise kleiner gleich 0,1% ist, wobei die Summe der Gehalte jedes dieser Nebenprodukte kleiner gleich 0,5% ist, wobei das Verfahren die folgenden aufeinanderfolgenden Schritte umfaßt:

- Kondensieren von Phthalsäureanhydrid mit Resorcin in einem Lösungsmittel, das aus einem Ester einer aliphatischen oder aromatischen organischen Säure, vorzugsweise Benzoesäure- oder Palmitinsäureethylester oder -methylester, besteht, in Gegenwart eines Katalysators ausgewählt aus der Gruppe, die insbesondere Lewis-Säuren oder Brönsted-Säuren und vorzugsweise ein Alkalimetallhydrogensulfat enthält,
- Suspendieren der aus dem vorhergehenden Schritt erhaltenen roten Kristalle in einem wasserfreien Lösungsmittel ausgewählt aus der Gruppe enthaltend Alkohole wie absolutes Ethanol, Ketone wie Aceton, Ether, halogenierte Lösungsmittel oder Mischungen davon,
- Ansäuern der so erhaltenen Suspension durch Zugabe einer starken Säure oder eines Vorläufers davon ausgewählt aus der Gruppe, die insbesondere Schwefelsäure, Salzsäure, Bromwasserstoffsäure, Fluorwasserstoffsäure, Iodwasserstoffsäure, Polyphosphorsäure, Pyrophosphat ($P_2O_5$) und Mischungen davon enthält, bis zur Umwandlung der roten Kristalle in gelbe Kristalle die das Röntgenkristallographiespektrum von Figur 2 aufweisen,
- Waschen der erhaltenen Kristalle mit Hilfe einer Waschlösung ausgewählt aus der Gruppe enthaltend Wasser, Alkohole, Ketone, Ether und halogenierte Lösungsmittel in reiner Form oder als Mischung, wobei das Waschen bis zur Rückumwandlung der gelben Kristalle in rote Kristalle fortgesetzt wird.

**11.** Verfahren nach einem der Ansprüche 7 bis 10, **dadurch gekennzeichnet, dass** man das Ansäuern durch Einleitung von gasförmiger Salzsäure in die Phthaleinsuspension oder durch Einwirkung auf dieses Phthalein von Salzsäure in Lösung in dem wasserfreien organischen Lösungsmittel, vorzugsweise einem Alkohol, einem Keton, einem Ether oder einem halogenierten Lösungsmittel für sich alleine oder als Mischung, noch weiter bevorzugt Isopropanol, absolutes Ethanol oder Aceton in reiner Form oder als Mischung durchgeführt wird.

**12.** Verfahren nach einem der Ansprüche 1 bis 11, **dadurch gekennzeichnet, dass** der für die Kondensationsreaktion verwendete Katalysator aus einem Hydrogensulfat eines Alkalimetalls, vorzugsweise Kalium- oder Natriumhydrogensulfat, besteht.

**13.** Fluoresceinkristalle gelber Farbe, die das Röntgenkristallographiespektrum mit den nachstehend angegebenen Peaks aufweisen:

| Theta (Grad) | Abstände (Å) | Intensitäten % | Impulsanzahl |
|---|---|---|---|
| 3,3500 | 13,1813 | 8,55 | 56 |
| 4,0050 | 11,0283 | 47,18 | 309 |
| 4,8550 | 9,1010 | 12,98 | 85 |
| 5,2650 | 8,3940 | 9,01 | 59 |
| 5,4500 | 8,1099 | 11,45 | 75 |
| 5,7900 | 7,6352 | 52,98 | 347 |
| 6,3000 | 7,0193 | 16,95 | 111 |
| 6,7700 | 6,5340 | 17,86 | 117 |
| 7,4900 | 5,9090 | 17,56 | 115 |

(fortgesetzt)

| Theta (Grad) | Abstände (Å) | Intensitäten % | Impulsanzahl |
|---|---|---|---|
| 8,0300 | 5,5140 | 69,77 | 457 |
| 8,4250 | 5,2572 | 33,13 | 217 |
| 8,7800 | 5,0462 | 8,40 | 55 |
| 9,1550 | 4,8411 | 15,88 | 104 |
| 9,3400 | 4,7461 | 42,44 | 278 |
| 9,6550 | 4,5926 | 34,66 | 227 |
| 9,8800 | 4,4891 | 24,43 | 160 |
| 10,1950 | 4,3518 | 57,71 | 378 |
| 10,5800 | 4,1951 | 58,02 | 380 |
| 10,7350 | 4,1352 | 38,17 | 250 |
| 11,1650 | 3,9779 | 28,85 | 189 |
| 11,4800 | 3,8701 | 30,23 | 198 |
| 11,7200 | 3,7919 | 59,85 | 392 |
| 12,1100 | 3,6716 | 26,41 | 173 |
| 12,3950 | 3,5884 | 58,47 | 383 |
| 12,6400 | 3,5200 | 100,00 | 655 |
| 12,9450 | 3,4384 | 30,84 | 202 |
| 13,1550 | 3,3845 | 56,64 | 371 |
| 13,8350 | 3,2211 | 14,66 | 96 |
| 14,0550 | 3,1717 | 24,12 | 158 |
| 14,3550 | 3,1068 | 36,34 | 238 |
| 14,6700 | 3,0415 | 42,90 | 281 |
| 15,1300 | 2,9511 | 42,90 | 281 |
| 15,7500 | 2,8377 | 18,47 | 121 |
| 15,9400 | 2,8047 | 18,17 | 119 |
| 16,2500 | 2,7526 | 15,42 | 101 |
| 16,7550 | 2,6719 | 17,71 | 116 |
| 16,8500 | 2,6573 | 19,39 | 127 |
| 17,2250 | 2,6011 | 18,17 | 119 |
| 17,7300 | 2,5293 | 15,73 | 103 |
| 18,4200 | 2,4377 | 10,23 | 67 |
| 18,5750 | 2,4180 | 10,23 | 67 |
| 19,2650 | 2,3345 | 15,88 | 104 |
| 19,5800 | 2,2984 | 10,84 | 71 |
| 19,8000 | 2,2739 | 11,91 | 78 |
| 20,1150 | 2,2397 | 13,28 | 87 |
| 20,6500 | 2,1841 | 14,81 | 97 |
| 21,1500 | 2,1348 | 11,30 | 74 |
| 21,2750 | 2,1228 | 10,08 | 66 |
| 21,8100 | 2,0732 | 7,94 | 52 |
| 22,2500 | 2,0342 | 9,16 | 60 |
| 22, 6250 | 2,0022 | 9, 62 | 63 |
| 22,9100 | 1,9786 | 9,01 | 59 |
| Wellenlänge: 1,54051 Å. | | | |

**14.** Fluoresceinkristalle, die gemäß einem Verfahren nach einem der Ansprüche 7 bis 11 erhältlich sind.

**15.** Verwendung der Fluoresceinkristalle nach Anspruch 14 bei pharmazeutischen Anwendungen in der Diagnostik, insbesondere der medizinischen bildlichen Darstellung, oder auf dem Gebiet der Biotechnologie-Anwendungen.

**FIGURE 1**

Radiocristallographie
de la fluorescéine brute

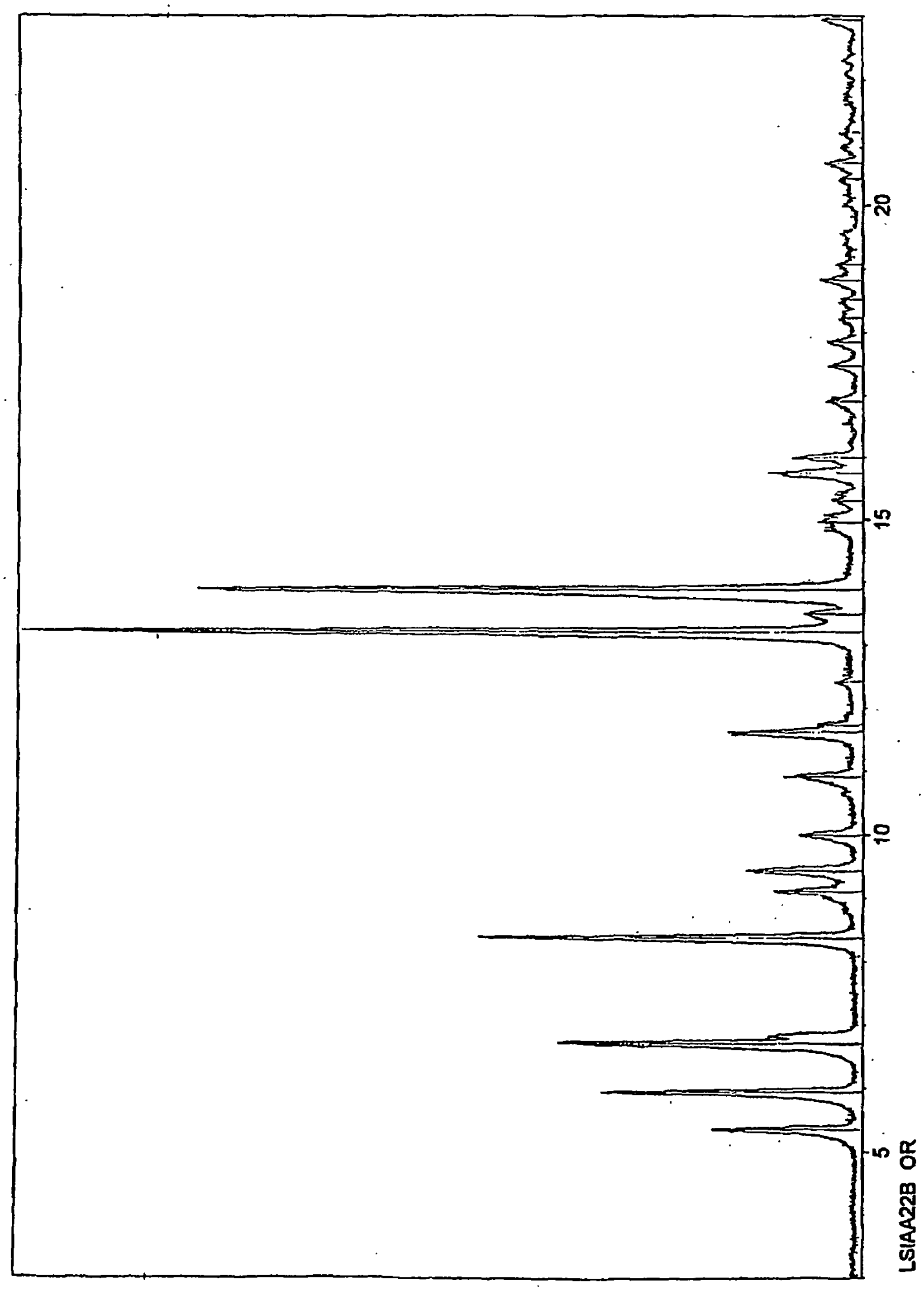

FIGURE 2

Radiocristallographie de la fluorescéine
après conversion par l'acide (jaune)

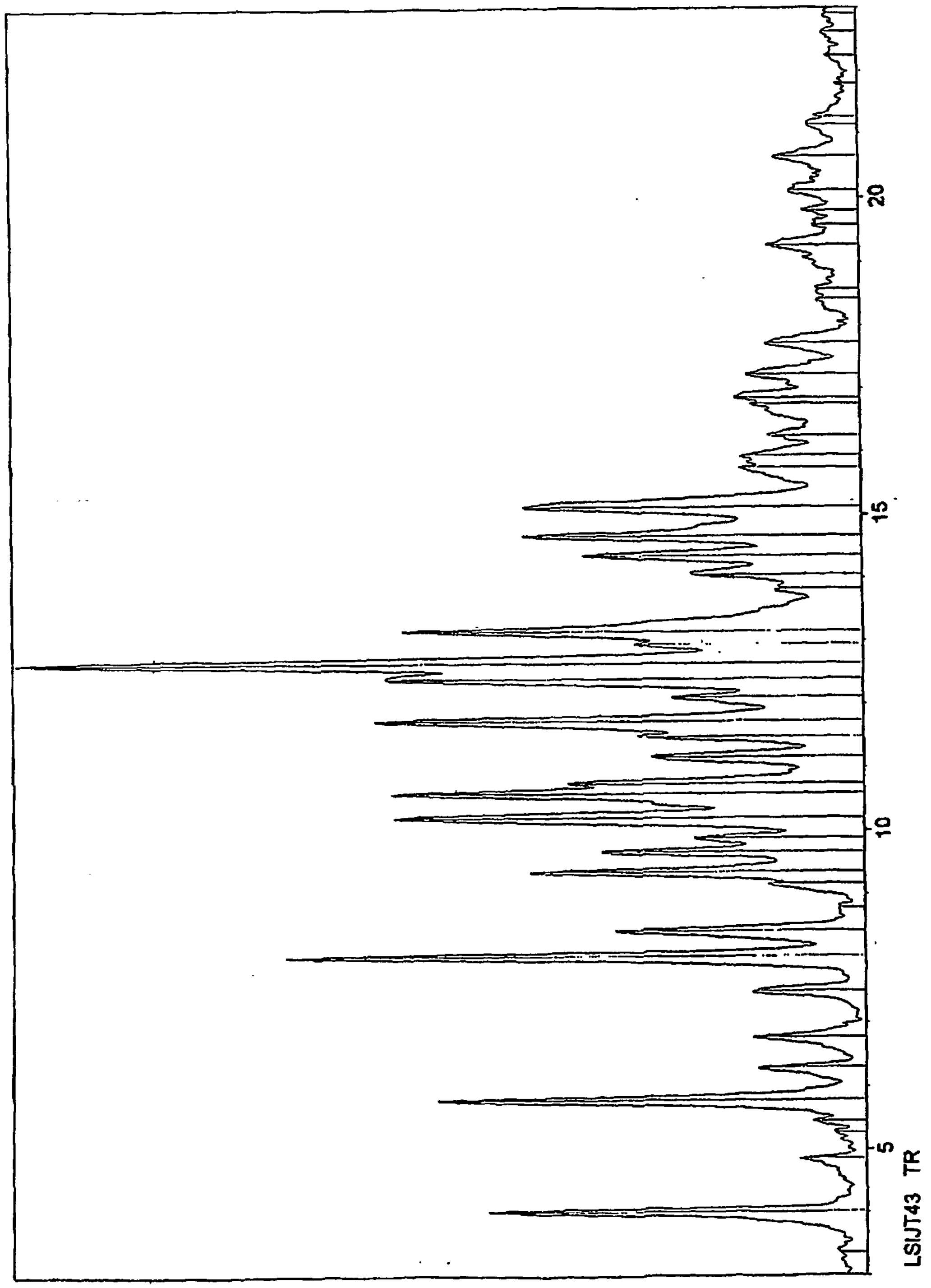

**FIGURE 3**

Radiocristallographie
de la 4',5'-diméthylfluorescéine brute

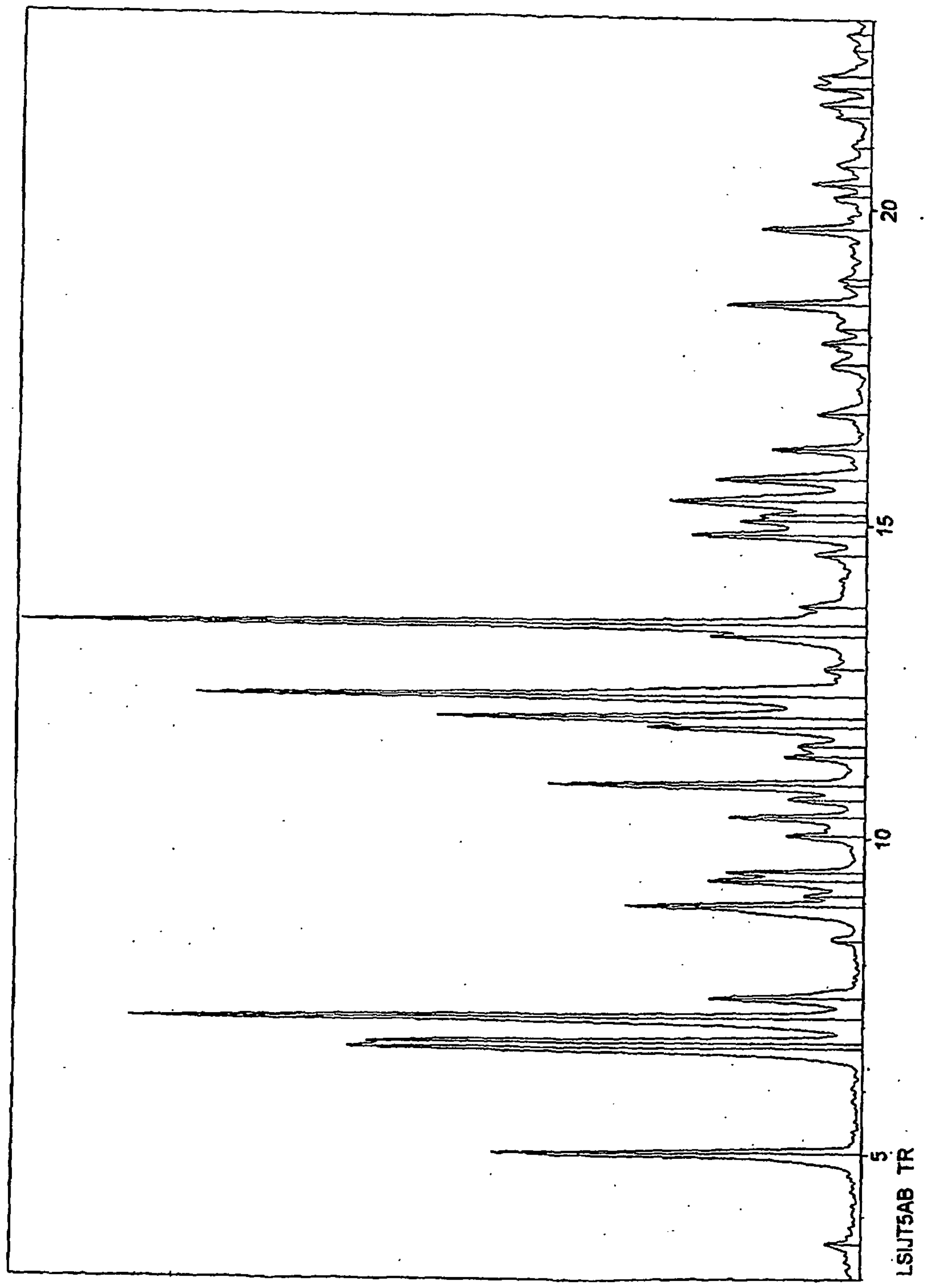

**FIGURE 4**

Radiocristallographie de la 4',5'-diméthylfluorescéine après conversion par l'acide

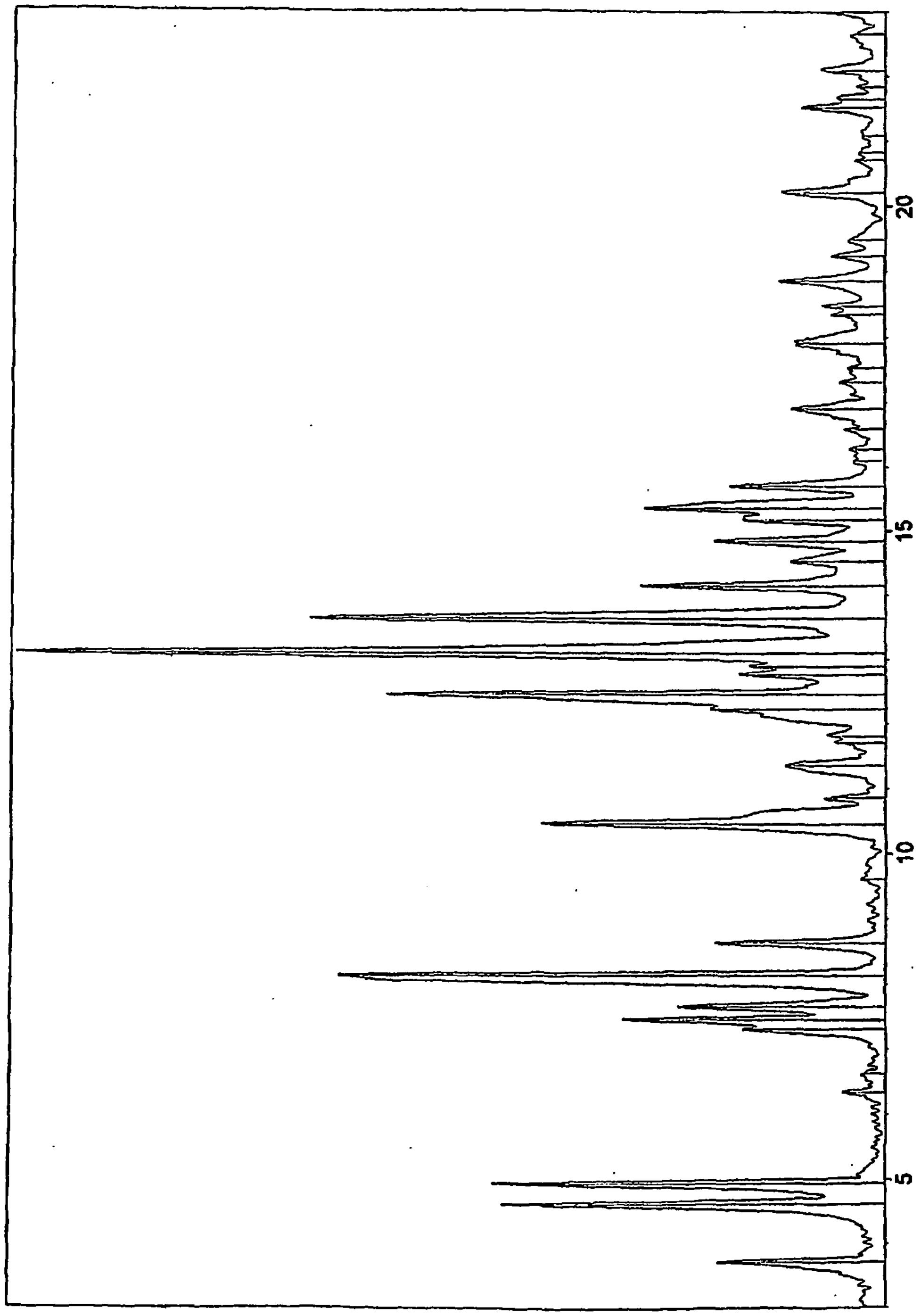

**FIGURE 5**

Radiocristallographie
de la 4',5'-dihydroxyfluorescéine brute

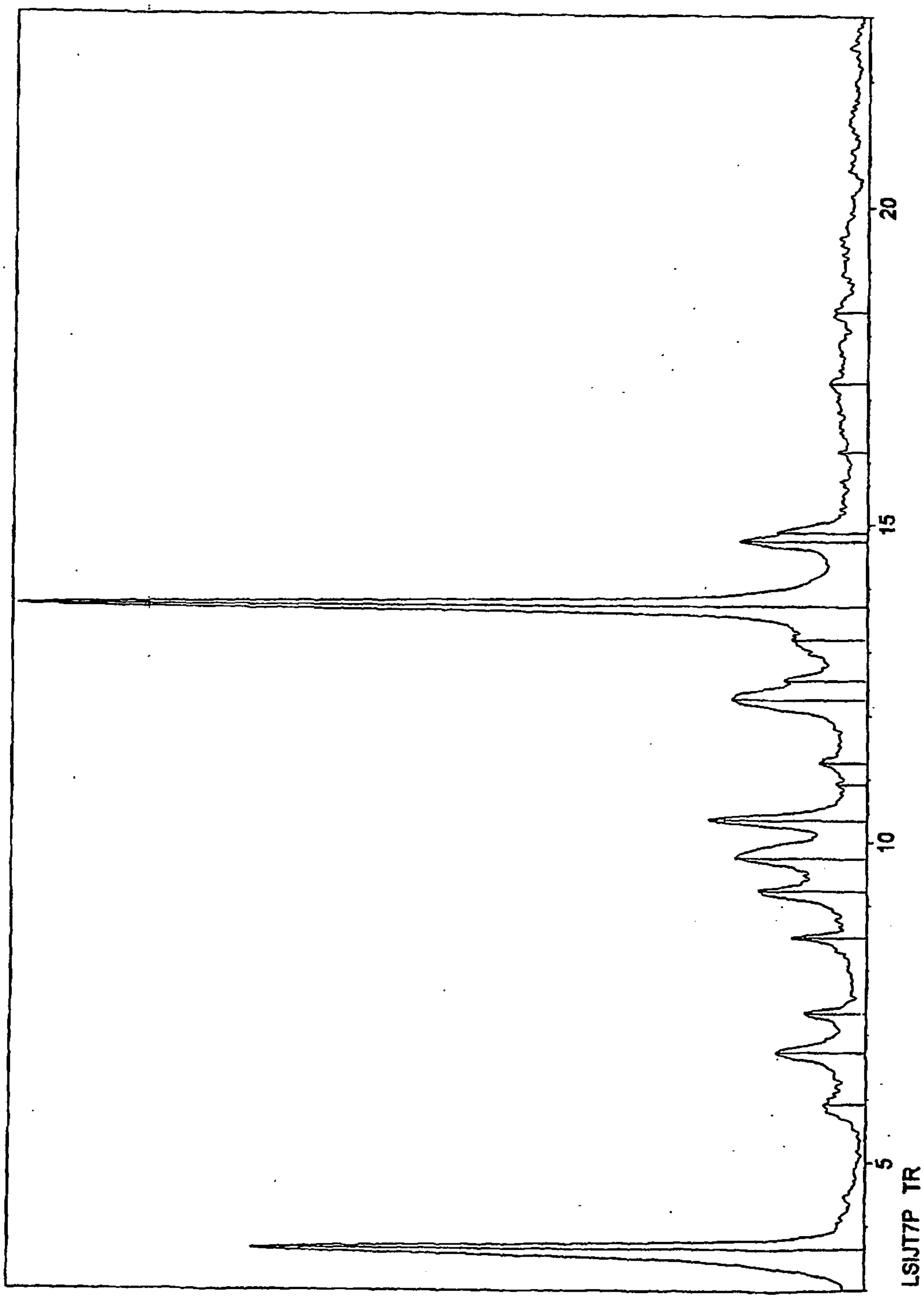

**FIGURE 6**

Radiocristallographie de la 4',5'-dihydroxyfluorescéine après conversion par l'acide

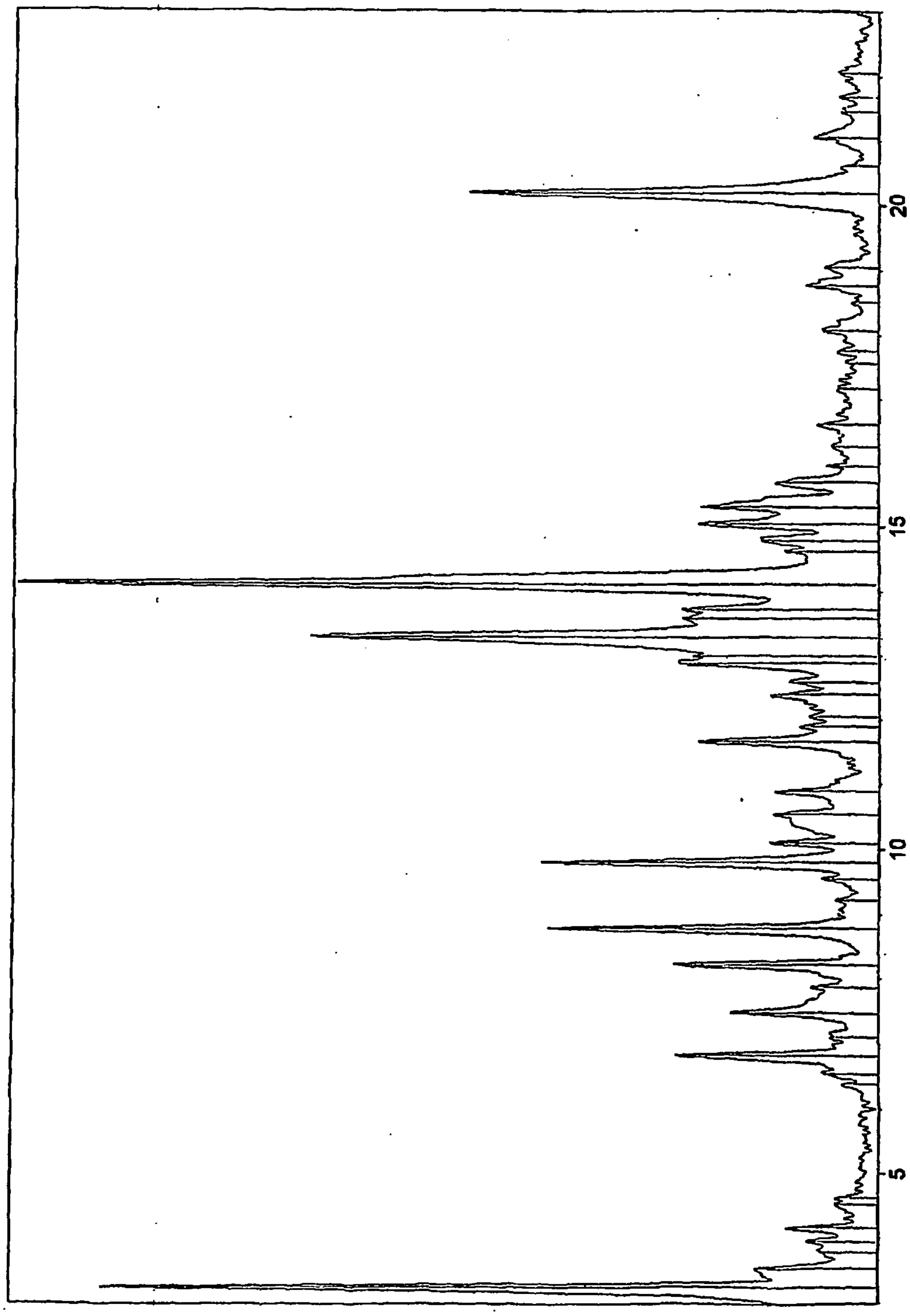

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5637733 A
- US 1931049 A
- DE 360691
- DD 136498
- US 1965842 A


### Littérature non-brevet citée dans la description


- ICH : International Commission of Harmonisation. *ICH Q Topic Q3A,* 1999
- **L. Yannuzzi.** Effective differences in the formulation of intravenous fluorescein and related side effects. *Am. J. Ophtalmol.,* 1974, vol. 78 (2), 217-221
- **Shawn C. Burdette et al.** *J.Am.Chem.Soc.,* 2001, vol. 123, 7831-41